(19) Europäisches Patentamt
European Patent Office
Office européen des brevets

(11) Veröffentlichungsnummer : **0 055 833**
**B1**

(12) # EUROPÄISCHE PATENTSCHRIFT

(45) Veröffentlichungstag der Patentschrift : **15.08.84**

(51) Int. Cl.³ : **C 07 D249/08**, C 07 D233/60

(21) Anmeldenummer : **81110222.7**

(22) Anmeldetag : **08.12.81**

(54) **Substituierte 1-Azolyl-butan-2-ole, Verfahren zu ihrer Herstellung und ihre Verwendung als Pflanzenschutzmittel sowie als Zwischenprodukte.**

(30) Priorität : **20.12.80 DE 3048267**

(43) Veröffentlichungstag der Anmeldung : **14.07.82 Patentblatt 82/28**

(45) Bekanntmachung des Hinweises auf die Patenterteilung : **15.08.84 Patentblatt 84/33**

(84) Benannte Vertragsstaaten : **AT BE CH DE FR GB IT LI NL SE**

(56) Entgegenhaltungen :
**EP-A- 0 032 200**
**DE-A- 2 908 378**
**DE-A- 2 920 437**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Krämer, Wolfgang, Dr.**
**Am Eckbusch 39/45**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Elbe, Hans-Ludwig, Dr.**
**Dasnoeckel 59**
**D-5600 Wuppertal 1 (DE)**
Erfinder : **Kraatz, Udo, Dr.**
**Körner Strasse 6**
**D-5090 Leverkusen 1 (DE)**
Erfinder : **Regel, Erik, Ing.-grad.**
**Bergerheide 72a**
**D-5600 Wuppertal 1 (DE)**
Erfinder : **Frohberger, Paul-Ernst, Dr.**
**Willi-Baumeister-Strasse 5**
**D-5090 Leverkusen 1 (DE)**
Erfinder : **Brandes, Wilhelm, Dr.**
**Eichendorffstrasse 3**
**D-5653 Leichlingen 1 (DE)**
Erfinder : **Lürssen, Klaus, Dr.**
**August-Kierspel-Strasse 89**
**D-5060 Bergisch-Gladbach 2 (DE)**

**EP 0 055 833 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte 1-Azolyl-butan-2-ole, ein Verfahren zu ihrer Herstellung sowie ihre Verwendung als Pflanzenschutzmittel und als Zwischenprodukte für die Synthese von weiteren Pflanzenschutzmitteln.

Es ist bereits bekannt geworden, daß bestimmte Triazolylketo- und -carbinol-Derivate, wie z. B. 3,3-Dimethyl-1-(1,2,4-triazol-1-yl)-butan-2-on und 1-(4-Chlorphenyl)-2-(1,2,4-triazol-1-yl)-propan-1-ol, allgemein gute fungizide Wirksamkeit besitzen (vergleiche DE-OS 2 431 407). Die Wirkung dieser Triazol-Derivate ist jedoch in bestimmten Indikationsbereichen, insbesondere bei niedrigen Aufwandmengen und -konzentrationen, nicht immer ganz befriedigend.

Es wurden neue substituierte 1-Azolyl-butan-2-ole der allgemeinen Formel I

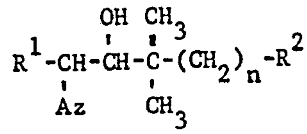

(I)

in welcher

Az für 1,2,4-Triazol-1-yl und -4-yl oder Imidazol-1-yl steht,

$R^1$ für Wasserstoff, Alkyl mit 1 bis 12 Kohlenstoffatomen, für Alkenyl und Alkinyl mit jeweils 2 bis 6 Kohlenstoffatomen steht, für gegebenenfalls durch Methyl substituiertes Cycloalkyl mit 3 bis 7 Kohlenstoffatomen oder Cycloalkylalkyl mit 3 bis 7 Kohlenstoffatomen in Cycloalkylteil und 1 bis 4 Kohlenstoffatomen im Alkylteil, sowie für Phenylalkyl und Phenoxyalkyl mit jeweils 1 bis 2 Kohlenstoffatomen im Alkylteil steht, wobei die Phenylalkyl- und Phenoxyalkyl-Gruppen im Phenylteil substituiert sein können durch Halogen, Alkyl mit 1 bis 6 Kohlenstoffatomen, durch Methoxy, Methylthio, Cyclohexyl, Dimethylamino, Trifluormethyl, Trifluormethoxy, Trifluormethylthio, durch Nitro, Cyano und Alkoxycarbonyl mit 1 bis 4 Kohlenstoffatomen im Alkylteil, sowie durch jeweils gegebenenfalls halogensubstituiertes Phenyl oder Phenoxy, sowie durch die Gruppierung —CO—$NR^6R^7$,

n für 0 oder 1 steht,

$R^2$ für Phenyl steht, welches durch die bei $R^1$ genannten Phenylsubstituenten gegebenenfalls substituiert sein kann, weiterhin für Cyano, für Alkoxycarbonyl mit 1 bis 4 Kohlenstoffatomen im Alkylteil und für die Gruppierungen —X—$R^3$ und —CO—$NR^4R^5$ steht, wobei

X für Sauerstoff, Schwefel, die SO- oder $SO_2$-Gruppe steht,

$R^3$ für Alkyl mit 1 bis 4 Kohlenstoffatomen, für Phenyl und für Benzyl steht, wobei die beiden letztgenannten Gruppen durch die bei $R^1$ genannten Phenylsubstituenten gegebenenfalls substituiert sein können,

$R^4$ für Wasserstoff, Alkyl mit 1 bis 4 Kohlenstoffatomen oder für Phenyl steht, wobei letzteres durch die bei $R^1$ genannten Phenylsubstituenten substituiert sein kann,

$R^5$ für Wasserstoff und Alkyl mit 1 bis 4 Kohlenstoffatomen steht, und

$R^6$ und $R^7$ für Wasserstoff, für Alkyl mit 1 bis 4 Kohlenstoffatomen und für gegebenenfalls durch Halogen substituiertes Phenyl stehen, oder beide zusammen mit dem verbindenden Stickstoff für einen Morpholinring stehen,

sowie deren pflanzenverträglichen Säureadditions-Salze und Metallsalz-Komplexe gefunden.

Die Verbindungen der allgemeinen Formel I besitzen gegebenenfalls zwei asymmetrische Kohlenstoffatome ; sie können dann in den beiden geometrischen Isomeren (threo- und erythro-Form) vorliegen, die in unterschiedlichen Mengenverhältnissen anfallen können. In beiden Fällen liegen sie als optische Isomeren vor. Sämtliche Isomeren werden erfindungsgemäß beansprucht.

Weiterhin wurde gefunden, daß man die substituierten 1-Azolyl-butan-2-ole der allgemeinen Formel I sowie deren pflanzenverträgliche Säureadditionssalze und Metallsalzkomplexe erhält, wenn man substituierte 1-Azolyl-butan-2-one der allgemeinen Formel II

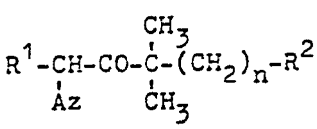

(II)

in welcher

Az, $R^1$, $R^2$ und n die oben angegebene Bedeutung haben,

nach bekannten Methoden in üblicher Weise reduziert und gegebenenfalls anschließend Säure oder ein Metallsalz addiert.

Die neuen substituierten 1-Azolyl-butan-2-ole der allgemeinen Formel weisen gute fungizide und pflanzenwachstumsregulierende Eigenschaften auf und sind daher als Pflanzenschutzmittel verwendbar.

Überraschenderweise zeigen die erfindungsgemäßen substituierten 1-Azolyl-butan-2-ole eine bessere fungizide Wirksamkeit als die aus dem Stand der Technik bekannten Triazol-Derivate 3,3-Dimethyl-1-(1,2,4-triazol-1-yl)-butan-2-on und 1-(4-Chlorphenyl)-2-(1,2,4-triazol-1-yl)-propan-1-ol, die chemisch und wirkungsmäßig naheliegende Verbindungen sind.

Außerdem sind die neuen substituierten 1-Azolyl-butan-2-ole interessante Zwischenprodukte zur Herstellung von weiteren Pflanzenschutz-Wirkstoffen. So können z. B. die Verbindungen der allgemeinen Formel I an der Hydroxy-Gruppe in üblicher Weise (z. B. durch die « Williamson'sche Ethersynthese ») in die entsprechenden Ether überführt werden. Weiterhin können durch Umsetzung mit z. B. Acylhalogeniden oder Carbamoylchloriden in prinzipiell bekannter Weise Acyl- oder Carbamoyl-Derivate der Verbindungen der allgemeinen Formel I erhalten werden.

Die erfindungsgemäßen Stoffe stellen somit eine wertvolle Bereicherung der Technik dar.

Die erfingungsgemäßen substituierten 1-Azolyl-butan-2-ole sind durch die Formel I allgemein definiert.

Bevorzugt sind diejenigen Verbindungen der allgemeinen Formel I, in denen

$R^1$ für geradkettiges oder verzweigtes Alkyl mit 1 bis 6 Kohlenstoffatomen, Alkenyl und Alkinyl mit jeweils 2 bis 6 Kohlenstoffatomen, gegebenenfalls durch Methyl substituiertes Cyclohexyl und Cyclohexylmethyl, sowie für gegebenenfalls substituiertes Phenoxyalkyl und gegebenenfalls substituiertes Phenylalkyl mit jeweils 1 bis 2 Kohlenstoffatomen im Alkylteil steht, wobei als Phenyl-Substituenten genannt seien : Fluor, Chlor, Methyl, Ethyl, Isopropyl, tert.-Butyl, Dimethylamino, Methoxy, Methylthio, Cyclohexyl, Trifluormethyl, Trifluormethoxy, Trifluormethylthio, Nitro, Cyano, sowie gegebenenfalls durch Fluor und Chlor substituiertes Phenyl und Phenoxy, sowie die Morpholinocarbonyl-, Phenylaminocarbonyl-, Chlorphenylaminocarbonyl- und Dibutylaminocarbonyl-Gruppe ;

$R^2$ für gegebenenfalls substituiertes Phenyl steht, wobei als Substituenten die bei $R^1$ bereits genannten Phenylsubstituenten infrage kommen, ferner für Cyano, Methoxycarbonyl, Ethoxycarbonyl, Isopropoxycarbonyl, sowie die Gruppierungen —X—$R^3$ und —CO—$NR^4R^5$ steht ;

$R^3$ für geradkettiges oder verzweigtes Alkyl mit 1 bis 4 Kohlenstoffatomen oder gegebenenfalls substituiertes Phenyl und Benzyl steht, wobei als Substituenten die bei $R^1$ bereits genannten Phenylsubstituenten infrage kommen ;

$R^4$ für Wasserstoff, Methyl, Ethyl, Isopropyl oder gegebenenfalls substituiertes Phenyl steht, wobei als Substituenten Halogen und Alkyl mit 1 bis 4 Kohlenstoffatomen infrage kommen ;

$R^5$ für Wasserstoff, Methyl oder Isopropyl steht ; und

Az, X und der Index n die in der Erfingungsdefinition angegebene Bedeutung haben.

Im einzelnen seien außer den bei den Herstellungsbeispielen genannten Verbindungen folgende Verbindungen der allgemeinen Formel I genannt (wobei Az für 1,2,4-Triazol-1-yl oder Imidazol-1-yl steht) :

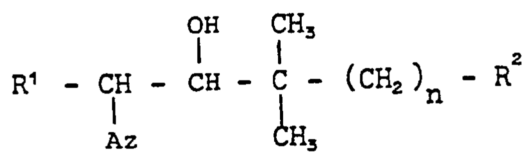

(I)

| $R^1$ | n | $R^2$ |
|---|---|---|
| H | 1 | 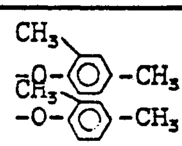 |
| $C_2H_5$ | 1 | |
| $C_4H_9$ | 1 | 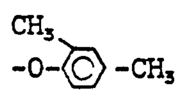 |
| 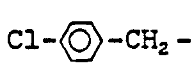 | 1 | 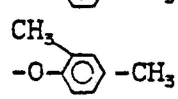 |
| 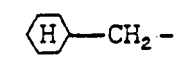 | 1 | 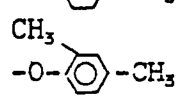 |
| H | 1 | 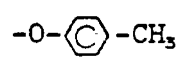 |
| $C_2H_5$ | 1 | 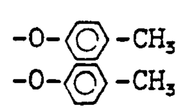 |
| $C_4H_9$ | 1 | |
| 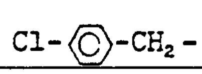 | 1 | 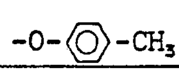 |

(Fortsetzung)

| $R^1$ | n | $R^2$ |
|---|---|---|
| 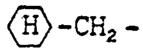 | 1 | 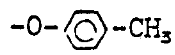 |
| H | 1 | 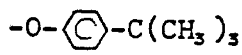 |
| $C_2H_5$ | 1 | 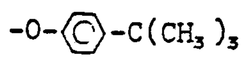 |
| $C_4H_9$ | 1 | 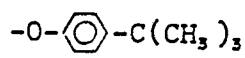 |
| 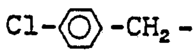 | 1 | 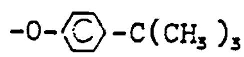 |
| 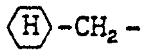 | 1 | 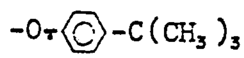 |
| H | 1 | 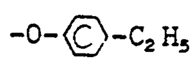 |
| $C_2H_5$ | 1 | 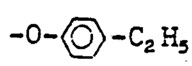 |
| $C_4H_9$ | 1 | 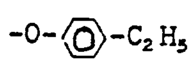 |
| 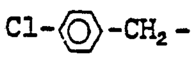 | 1 | 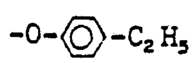 |
| 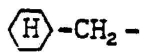 | 1 | 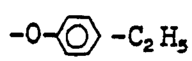 |
| H | 1 | 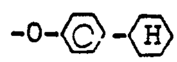 |
| $C_2H_5$ | 1 | 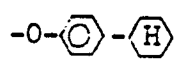 |
| $C_4H_9$ | 1 | 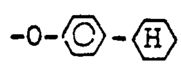 |
| 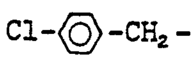 | 1 | 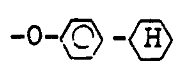 |
| 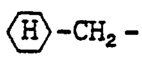 | 1 | 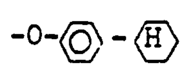 |
| H | 1 | 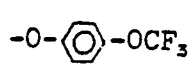 |
| $C_2H_5$ | 1 | 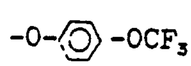 |
| $C_4H_9$ | 1 | 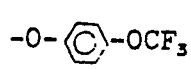 |
| 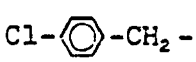 | 1 | 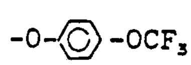 |
| 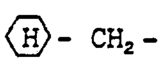 | 1 | 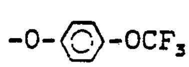 |

(Fortsetzung)

| $R^1$ | n | $R^2$ |
|---|---|---|
| H | 1 | 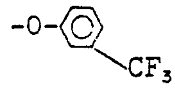 |
| $C_2H_5$ | 1 | 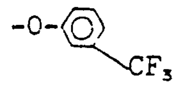 |
| $C_4H_9$ | 1 | 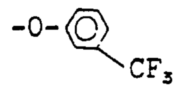 |
| 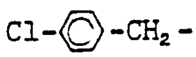 | 1 | 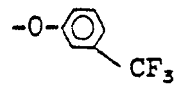 |
| 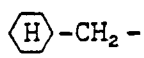 | 1 | 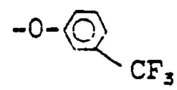 |
| H | 1 | 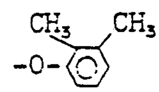 |
| $C_2H_5$ | 1 | 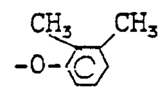 |
| $C_4H_9$ | 1 | 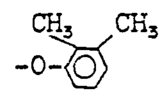 |
| 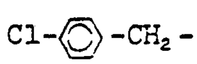 | 1 | 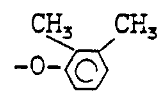 |
| 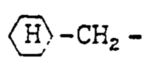 | 1 | 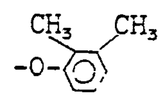 |
| H | 1 | 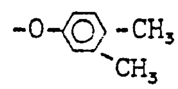 |
| $C_2H_5$ | 1 | 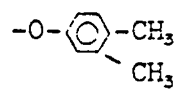 |
| $C_4H_9$ | 1 | 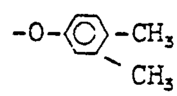 |
| 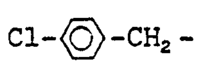 | 1 | 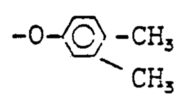 |
| 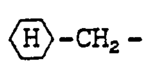 | 1 | 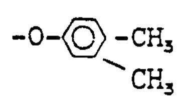 |

(Fortsetzung)

| $R^1$ | n | $R^2$ |
|---|---|---|
| H | 1 | 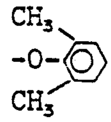 |
| $C_2H_5$ | 1 | 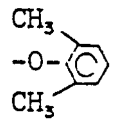 |
| $C_4H_9$ | 1 | 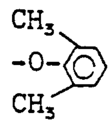 |
| 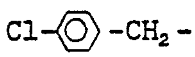 | 1 | 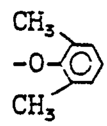 |
| 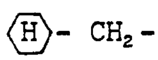 | 1 | 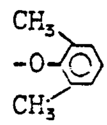 |
| H | 1 | 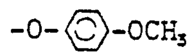 |
| $C_2H_5$ | 1 | 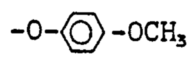 |
| $C_4H_9$ | 1 | 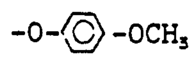 |
| 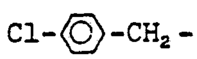 | 1 | 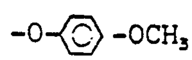 |
| 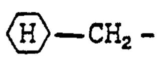 | 1 | 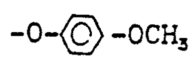 |
| H | 1 | 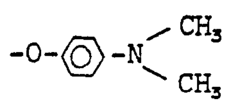 |
| $C_2H_5$ | 1 | 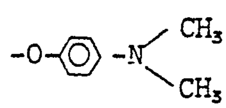 |
| $C_4H_9$ | 1 | 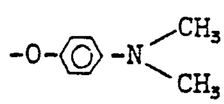 |
| 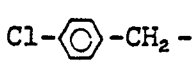 | 1 | 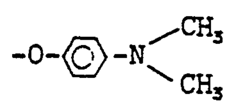 |
| 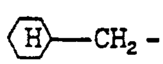 | 1 | 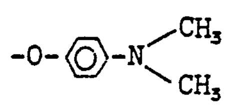 |
| H | 1 | 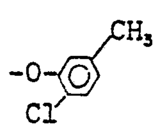 |

(Fortsetzung)

| $R^1$ | n | $R^2$ |
|---|---|---|
| $C_2H_5$ | 1 | 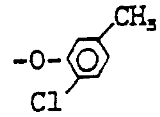 |
| $C_4H_9$ | 1 | 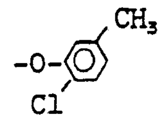 |
| 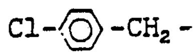 | 1 | 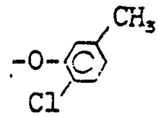 |
| 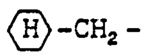 | 1 | 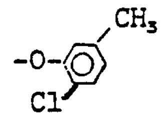 |
| H | 1 | 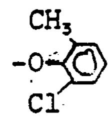 |
| $C_2H_5$ | 1 | 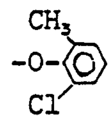 |
| $C_4H_9$ | 1 | 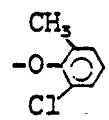 |
| 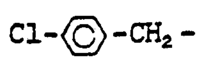 | 1 | 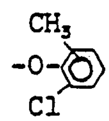 |
| 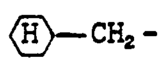 | 1 | 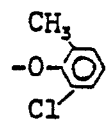 |
| H | 1 | 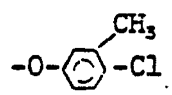 |
| $C_2H_5$ | 1 | 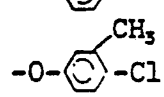 |
| $C_4H_9$ | 1 | 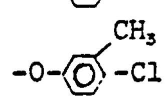 |
| 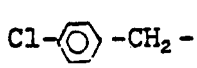 | 1 | 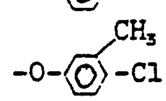 |
| 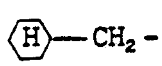 | 1 | 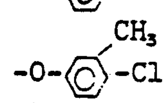 |
| H | 1 | 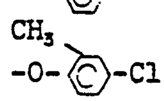 |
| $C_2H_5$ | 1 | 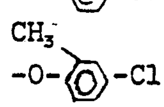 |

(Fortsetzung)

| $R^1$ | n | $R^2$ |
|---|---|---|
| $C_4H_9$ | 1 | 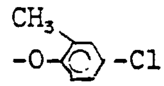 |
| 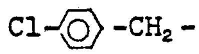 | 1 | 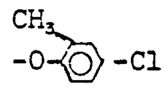 |
| 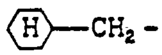 | 1 | 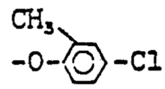 |
| H | 1 | 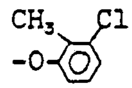 |
| $C_2H_5$ | 1 | 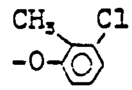 |
| $C_4H_9$ | 1 | 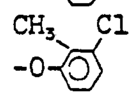 |
| 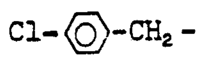 | 1 | 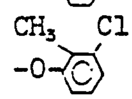 |
| 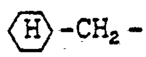 | 1 | 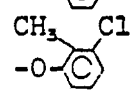 |
| H | 1 | 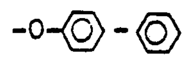 |
| $C_2H_5$ | 1 | 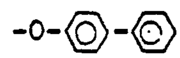 |
| $C_4H_9$ | 1 | 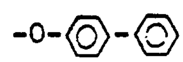 |
| 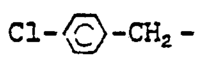 | 1 | 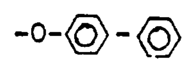 |
| 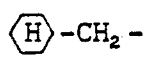 | 1 | 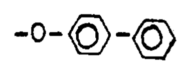 |
| H | 1 | 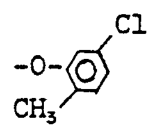 |
| $C_2H_5$ | 1 | 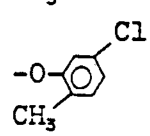 |
| $C_4H_9$ | 1 | 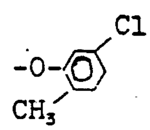 |
| 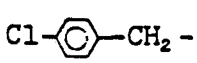 | 1 | 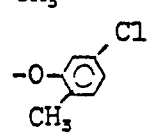 |
| 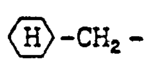 | 1 | 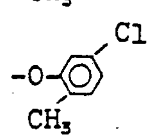 |

(Fortsetzung)

| R¹ | n | R² |
|---|---|---|
| H | 1 | 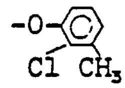 |
| $C_2H_5$ | 1 | 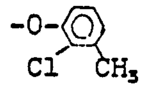 |
| $C_4H_9$ | 1 | 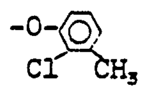 |
| 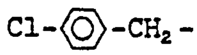 | 1 | 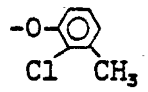 |
| 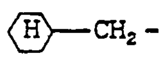 | 1 | 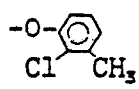 |
| H | 1 | 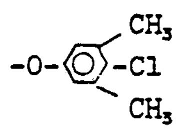 |
| $C_2H_5$ | 1 | 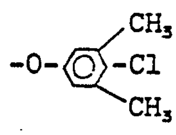 |
| $C_4H_9$ | 1 | 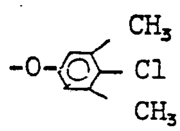 |
| 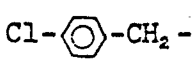 | 1 | 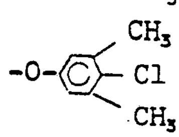 |
| 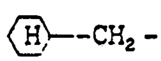 | 1 | 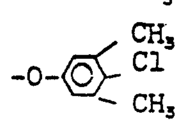 |
| H | 1 | 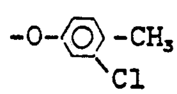 |
| $C_2H_5$ | 1 | 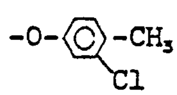 |
| $C_4H_9$ | 1 | 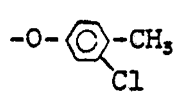 |
| 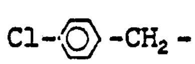 | 1 | 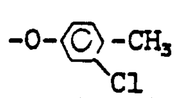 |

(Fortsetzung)

| $R^1$ | n | $R^2$ |
|---|---|---|
| 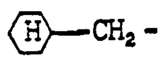 | 1 | 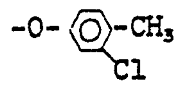 |
| H | 1 | 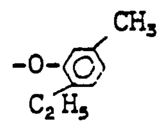 |
| $C_2H_5$ | 1 | 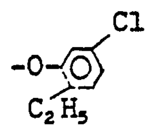 |
| $C_4H_9$ | 1 | 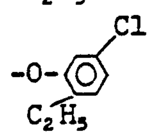 |
| 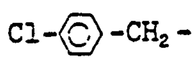 | 1 | 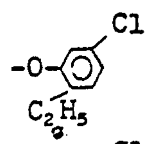 |
| 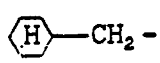 | 1 | 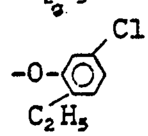 |
| H | 1 | 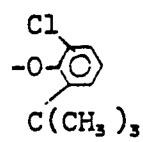 |
| $C_2H_5$ | 1 | 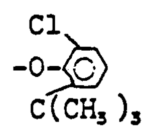 |
| $C_4H_9$ | 1 | 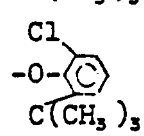 |
| 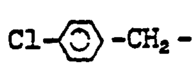 | 1 | 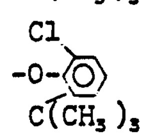 |
| 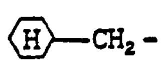 | 1 | 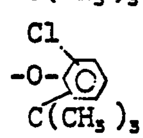 |
| H | 1 | 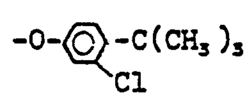 |
| $C_2H_5$ | 1 | 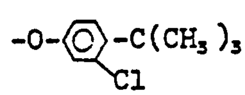 |
| $C_4H_9$ | 1 | 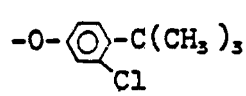 |

(Fortsetzung)

| $R^1$ | n | $R^2$ |
|---|---|---|
| 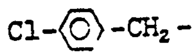 | 1 | 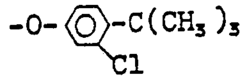 |
| 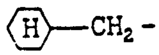 | 1 | 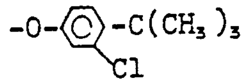 |
| H | 1 | 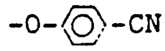 |
| $C_2H_5$ | 1 | 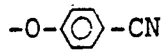 |
| $C_4H_9$ | 1 | 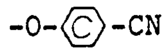 |
| 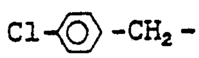 | 1 | 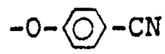 |
| 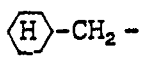 | 1 | 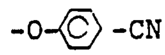 |
| H | 1 | 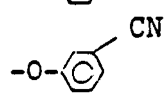 |
| $C_2H_5$ | 1 | 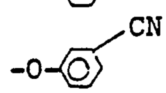 |
| $C_4H_9$ | 1 | 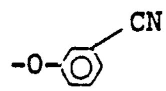 |
| 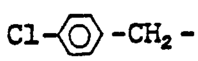 | 1 | 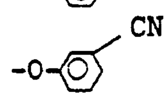 |
| 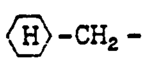 | 1 | 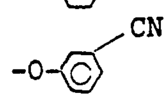 |
| H | 1 | 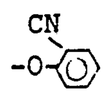 |
| $C_2H_5$ | 1 | 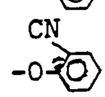 |
| $C_4H_9$ | 1 | 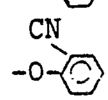 |
| 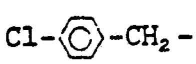 | 1 | 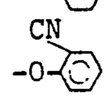 |
| 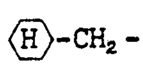 | 1 | 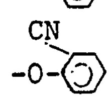 |
| H | 1 | 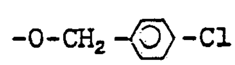 |
| $C_2H_5$ | 1 | 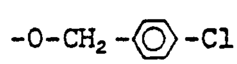 |
| $C_4H_9$ | 1 | 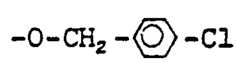 |

(Fortsetzung)

| $R^1$ | n | $R^2$ |
|---|---|---|
| 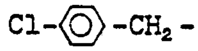 | 1 | 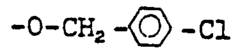 |
| 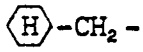 | 1 | 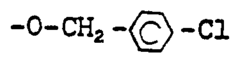 |
| H | 1 | 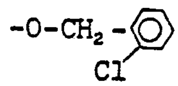 |
| $C_2H_5$ | 1 | 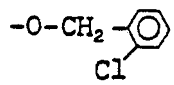 |
| $C_4H_9$ | 1 | 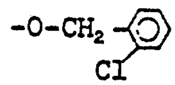 |
| 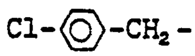 | 1 | 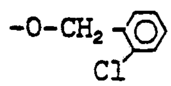 |
| 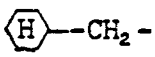 | 1 | 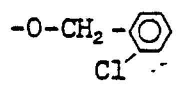 |
| H | 1 | 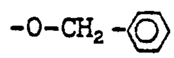 |
| $C_2H_5$ | 1 | 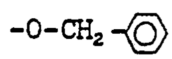 |
| $C_4H_9$ | 1 | 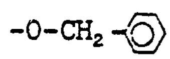 |
| 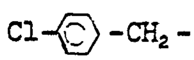 | 1 | 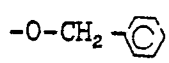 |
| 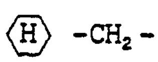 | 1 | 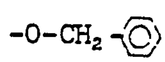 |
| H | 1 | 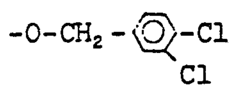 |
| $C_2H_5$ | 1 | 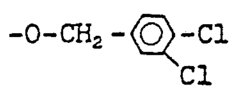 |
| $C_4H_9$ | 1 | 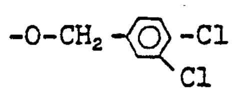 |
| 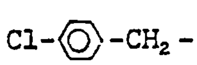 | 1 | 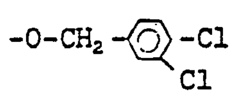 |
| 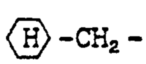 | 1 | 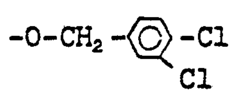 |

(Fortsetzung)

| $R^1$ | n | $R^2$ |
|---|---|---|
| H | 1 | 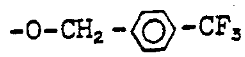 |
| $C_2H_5$ | 1 | 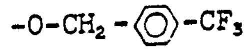 |
| $C_4H_9$ | 1 | 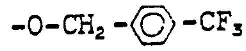 |
| 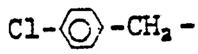 | 1 | 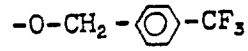 |
| 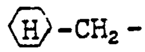 | 1 | 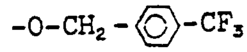 |
| H | 1 | 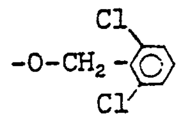 |
| $C_2H_5$ | 1 | 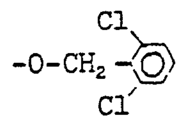 |
| $C_4H_9$ | 1 | 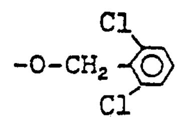 |
| 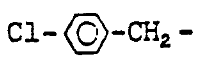 | 1 | 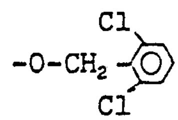 |
| 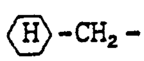 | 1 | 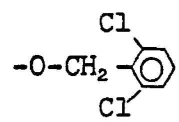 |
| H | 1 | 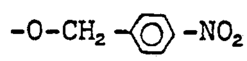 |
| $C_2H_5$ | 1 | 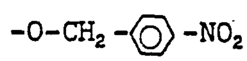 |
| $C_4H_9$ | 1 | 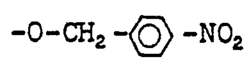 |
| 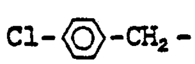 | 1 | 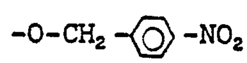 |
| 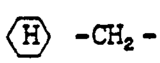 | 1 | 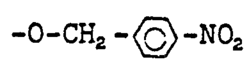 |
| H | 1 | 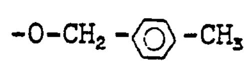 |
| $C_2H_5$ | 1 | 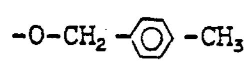 |
| $C_4H_9$ | 1 | 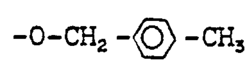 |

(Fortsetzung)

| $R^1$ | n | $R^2$ |
|---|---|---|
| 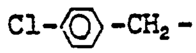 | 1 | 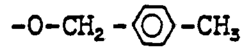 |
| 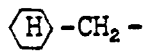 | 1 | 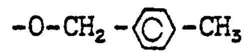 |
| H | 1 | 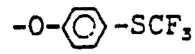 |
| $C_2H_5$ | 1 | 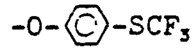 |
| $C_4H_9$ | 1 | 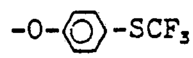 |
| 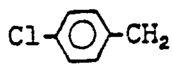 | 1 | 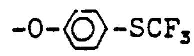 |
| 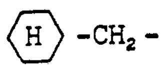 | 1 | 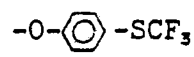 |
| H | 1 | 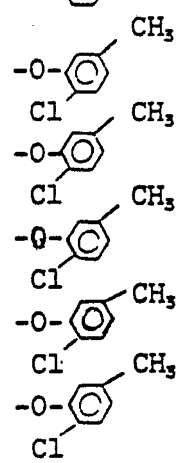 |
| $C_2H_5$ | 1 | |
| $C_4H_9$ | 1 | |
| 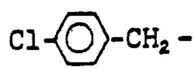 | 1 | |
| 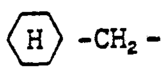 | 1 | |
| H | 0 | 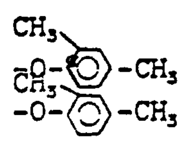 |
| $C_2H_5$ | 0 | |
| $C_4H_9$ | 0 | 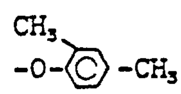 |
| 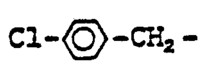 | 0 | 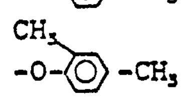 |
| 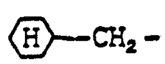 | 0 | 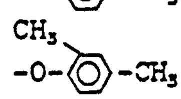 |
| H | 0 | 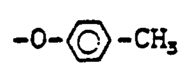 |
| $C_2H_5$ | 0 | 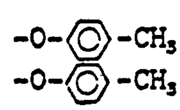 |
| $C_4H_9$ | 0 | |
| 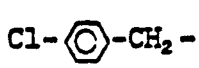 | 0 | 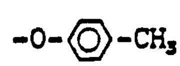 |

(Fortsetzung)

| $R^1$ | n | $R^2$ |
|---|---|---|
| 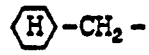 | 0 | 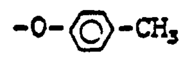 |
| H | 0 | 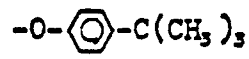 |
| $C_2H_5$ | 0 | 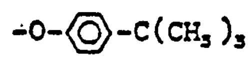 |
| $C_4H_9$ | 0 | 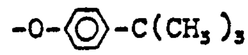 |
| 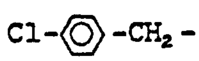 | 0 | 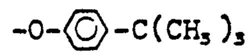 |
| 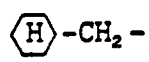 | 0 | 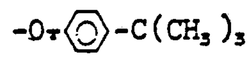 |
| H | 0 | 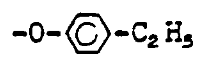 |
| $C_2H_5$ | 0 | 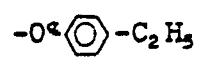 |
| $C_4H_9$ | 0 | 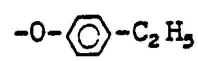 |
| 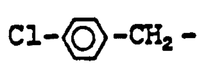 | 0 | 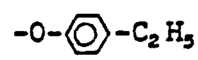 |
| 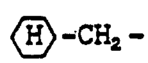 | 0 | 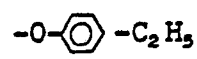 |
| H | 0 | 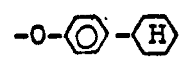 |
| $C_2H_5$ | 0 | 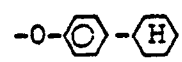 |
| $C_4H_9$ | 0 | 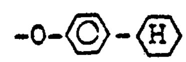 |
| 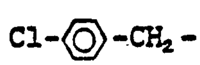 | 0 | 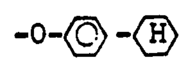 |
| 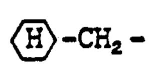 | 0 | 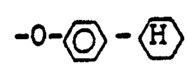 |
| H | 0 | 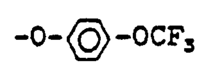 |
| $C_2H_5$ | 0 | 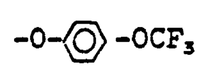 |
| $C_4H_9$ | 0 | 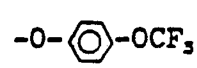 |
| 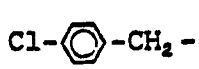 | 0 | 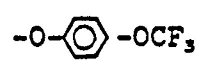 |
| 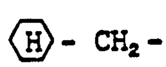 | 0 | 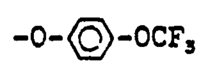 |

(Fortsetzung)

| $R^1$ | n | $R^2$ |
|---|---|---|
| H | 0 | 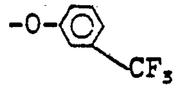 |
| $C_2H_5$ | 0 | 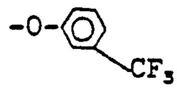 |
| $C_4H_9$ | 0 | 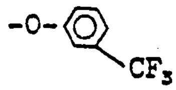 |
| 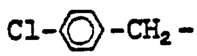 | 0 | 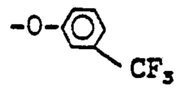 |
| 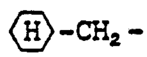 | 0 | 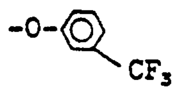 |
| H | 0 | 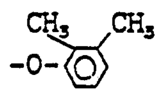 |
| $C_2H_5$ | 0 | 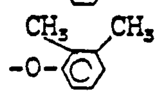 |
| $C_4H_9$ | 0 | 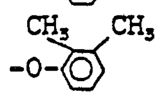 |
| 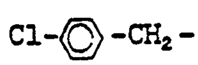 | 0 | 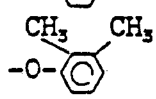 |
| 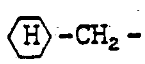 | 0 | 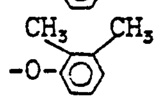 |
| H | 0 | 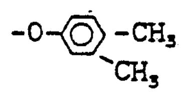 |
| $C_2H_5$ | 0 | 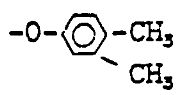 |
| $C_4H_9$ | 0 | 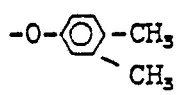 |
| 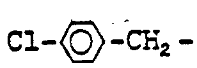 | 0 | 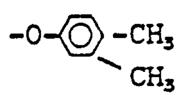 |
| 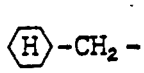 | 0 | 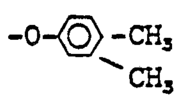 |
| H | 0 | 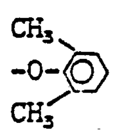 |

(Fortsetzung)

| $R^1$ | n | $R^2$ |
|---|---|---|
| $C_2H_5$ | 0 | 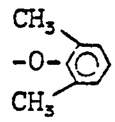 |
| $C_4H_9$ | 0 | 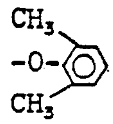 |
| 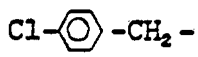 | 0 | 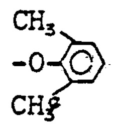 |
| 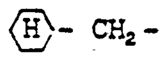 | 0 | 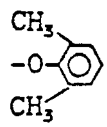 |
| H | 0 | 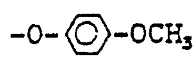 |
| $C_2H_5$ | 0 | 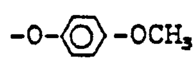 |
| $C_4H_9$ | 0 | 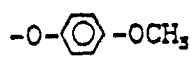 |
| 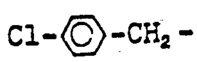 | 0 | 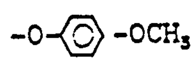 |
| 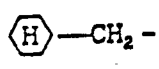 | 0 | 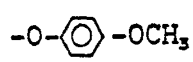 |
| H | 0 | 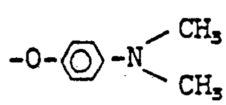 |
| $C_2H_5$ | 0 | 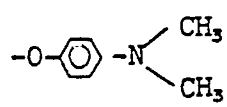 |
| $C_4H_9$ | 0 | 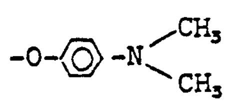 |
| 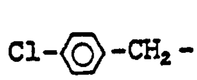 | 0 | 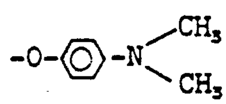 |
| 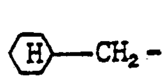 | 0 | 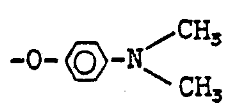 |
| H | 0 | 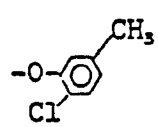 |
| $C_2H_5$ | 0 | 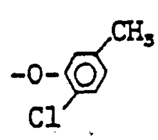 |

(Fortsetzung)

| $R^1$ | n | $R^2$ |
|---|---|---|
| $C_4H_9$ | 0 | 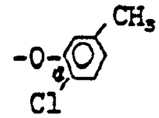 |
| 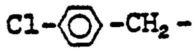 | 0 | 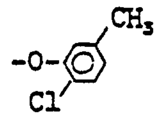 |
| 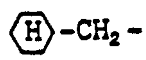 | 0 | 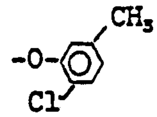 |
| H | 0 | 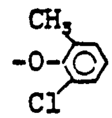 |
| $C_2H_5$ | 0 | 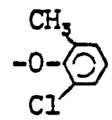 |
| $C_4H_9$ | 0 | 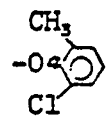 |
| 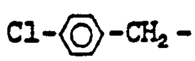 | 0 | 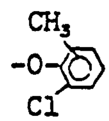 |
| 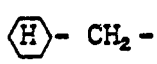 | 0 | 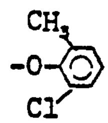 |
| H | 0 | 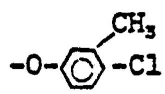 |
| $C_2H_5$ | 0 | 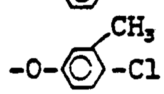 |
| $C_4H_9$ | 0 | 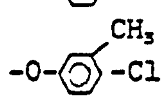 |
| 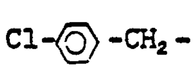 | 0 | 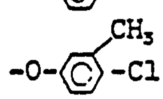 |
| 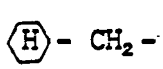 | 0 | 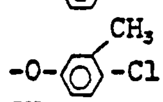 |
| H | 0 | 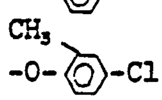 |
| $C_2H_5$ | 0 | 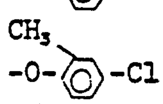 |
| $C_4H_9$ | 0 | 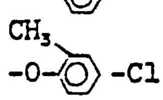 |

(Fortsetzung)

| $R^1$ | n | $R^2$ |
|---|---|---|
| 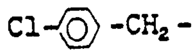 | 0 | 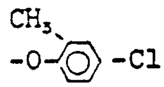 |
| 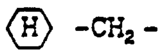 | 0 | 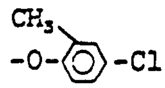 |
| H | 0 | 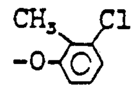 |
| $C_2H_5$ | 0 | 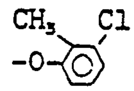 |
| $C_4H_9$ | 0 | 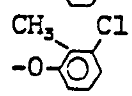 |
| 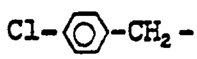 | 0 | 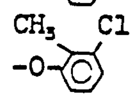 |
| 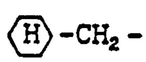 | 0 | 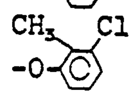 |
| H | 0 | 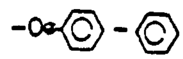 |
| $C_2H_5$ | 0 | 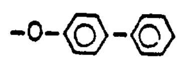 |
| $C_4H_9$ | 0 | 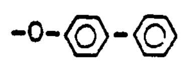 |
| 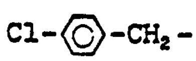 | 0 | 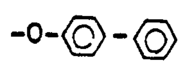 |
| 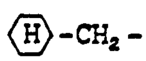 | 0 | 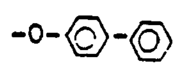 |
| H | 0 | 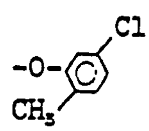 |
| $C_2H_5$ | 0 | 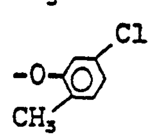 |
| $C_4H_9$ | 0 | 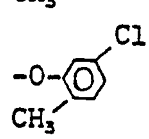 |
| 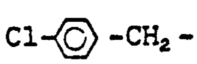 | 0 | 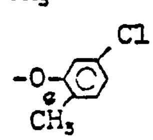 |
| 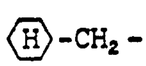 | 0 | 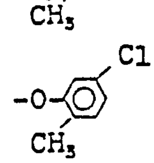 |

(Fortsetzung)

| $R^1$ | n | $R^2$ |
|---|---|---|
| H | 0 | 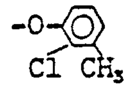 |
| $C_2H_5$ | 0 | 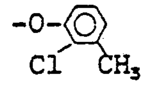 |
| $C_4H_9$ | 0 | 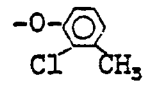 |
| 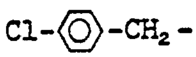 | 0 | 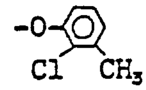 |
| 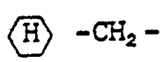 | 0 | 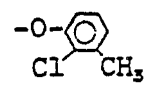 |
| H | 0 | 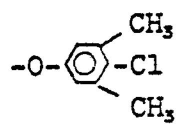 |
| $C_2H_5$ | 0 | 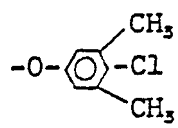 |
| $C_4H_9$ | 0 | 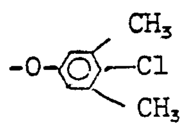 |
| 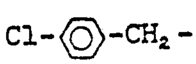 | 0 | 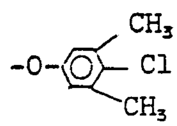 |
| 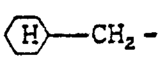 | 0 | 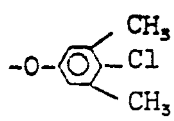 |
| H | 0 | 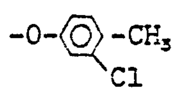 |
| $C_2H_5$ | 0 | 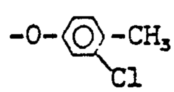 |
| $C_4H_9$ | 0 | 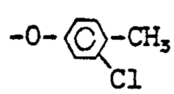 |
| 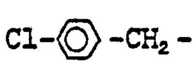 | 0 | 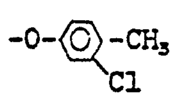 |

(Fortsetzung)

| $R^1$ | n | $R^2$ |
|---|---|---|
| 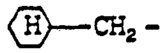 | 0 | 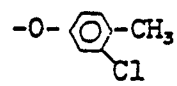 |
| H | 0 | 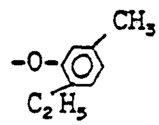 |
| $C_2H_5$ | 0 | 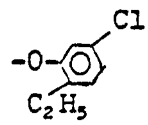 |
| $C_4H_9$ | 0 | 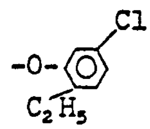 |
| 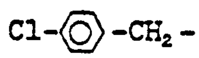 | 0 | 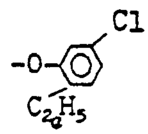 |
| 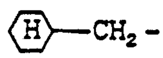 | 0 | 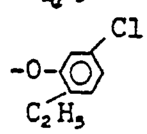 |
| H | 0 | 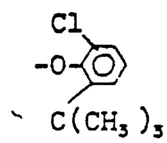 |
| $C_2H_5$ | 0 | 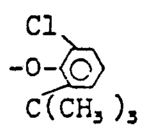 |
| $C_4H_9$ | 0 | 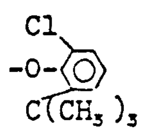 |
| 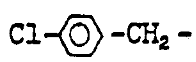 | 0 | 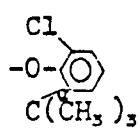 |
| 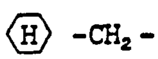 | 0 | 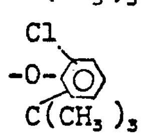 |
| H | 0 | 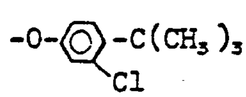 |
| $C_2H_5$ | 0 | 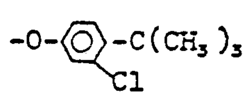 |
| $C_4H_9$ | 0 | 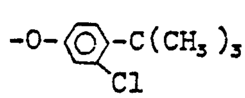 |

(Fortsetzung)

| $R^1$ | n | $R^2$ |
|---|---|---|
| 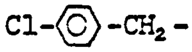 | 0 | 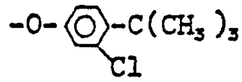 |
| 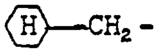 | 0 | 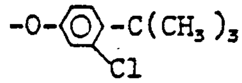 |
| H | 0 | 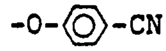 |
| $C_2H_5$ | 0 | 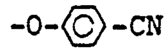 |
| $C_4H_9$ | 0 | 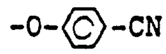 |
| 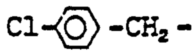 | 0 | 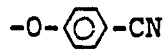 |
| 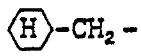 | 0 | 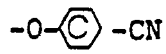 |
| H | 0 | 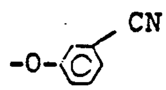 |
| $C_2H_5$ | 0 | 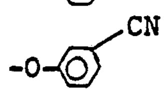 |
| $C_4H_9$ | 0 | 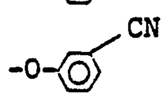 |
| 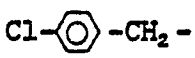 | 0 | 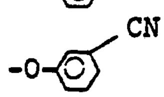 |
| 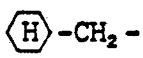 | 0 | 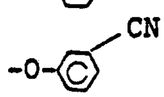 |
| H | 0 | 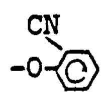 |
| $C_2H_5$ | 0 | 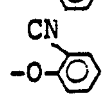 |
| $C_4H_9$ | 0 | 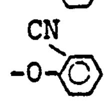 |
| 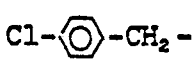 | 0 | 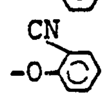 |
| 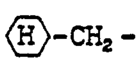 | 0 | 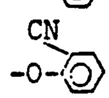 |
| H | 0 | 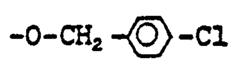 |
| $C_2H_5$ | 0 | 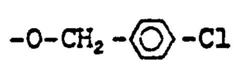 |
| $C_4H_9$ | 0 | 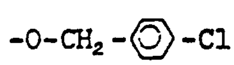 |

(Fortsetzung)

| $R^1$ | n | $R^2$ |
|---|---|---|
| 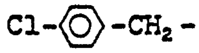 | 0 | 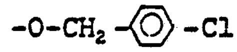 |
| 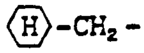 | 0 | 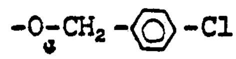 |
| H | 0 | 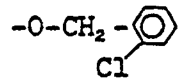 |
| $C_2H_5$ | 0 | 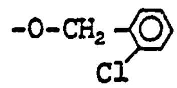 |
| $C_4H_9$ | 0 | 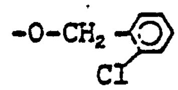 |
| 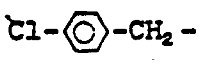 | 0 | 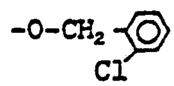 |
| 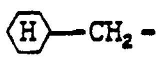 | 0 | 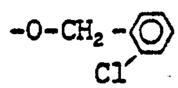 |
| H | 0 | 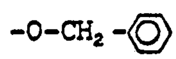 |
| $C_2H_5$ | 0 | 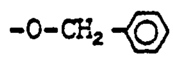 |
| $C_4H_9$ | 0 | 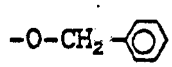 |
| 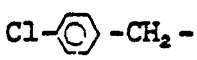 | 0 | 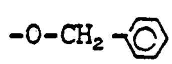 |
| 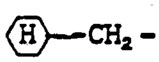 | 0 | 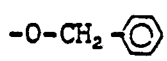 |
| H | 0 | 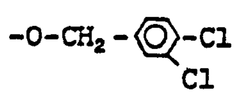 |
| $C_2H_5$ | 0 | 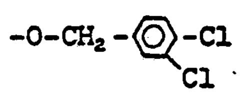 |
| $C_4H_9$ | 0 | 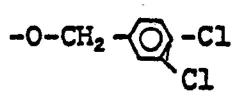 |
| 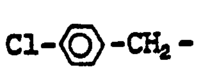 | 0 | 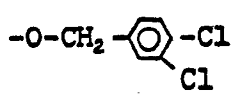 |
| 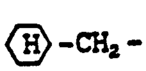 | 0 | 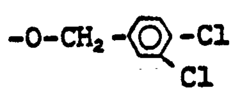 |

(Fortsetzung)

| $R^1$ | n | $R^2$ |
|---|---|---|
| H | 0 | 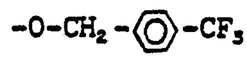 |
| $C_2H_5$ | 0 | 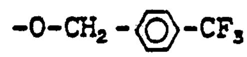 |
| $C_4H_9$ | 0 | 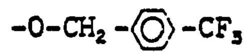 |
| 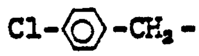 | 0 | 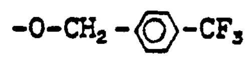 |
| 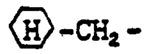 | 0 | 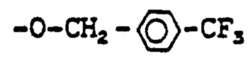 |
| H | 0 | 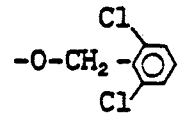 |
| $C_2H_5$ | 0 | 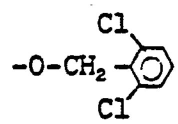 |
| $C_4H_9$ | 0 | 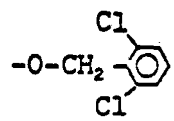 |
| 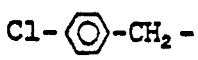 | 0 | 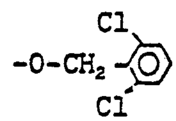 |
| 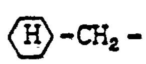 | 0 | 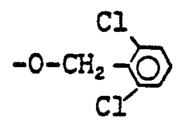 |
| H | 0 | 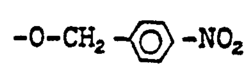 |
| $C_2H_5$ | 0 | 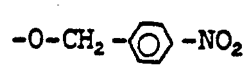 |
| $C_4H_9$ | 0 | 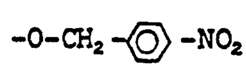 |
| 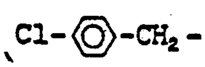 | 0 | 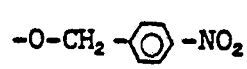 |
| 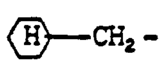 | 0 | 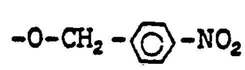 |
| H | 0 | 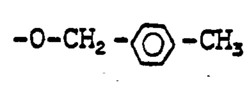 |
| $C_2H_5$ | 0 | 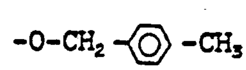 |
| $C_4H_9$ | 0 | 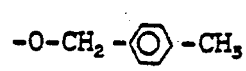 |

(Fortsetzung)

| $R^1$ | n | $R^2$ |
|---|---|---|
| 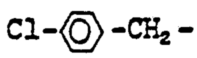 | 0 | 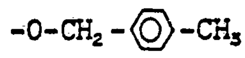 |
| 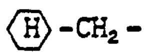 | 0 | 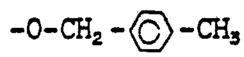 |
| H | 0 | 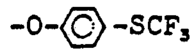 |
| $C_2H_5$ | 0 | 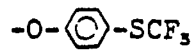 |
| $C_4H_9$ | 0 | 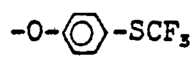 |
| 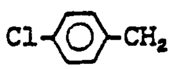 | 0 | 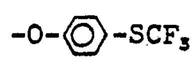 |
| 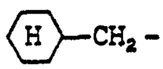 | 0 | 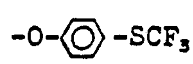 |
| H | 0 | 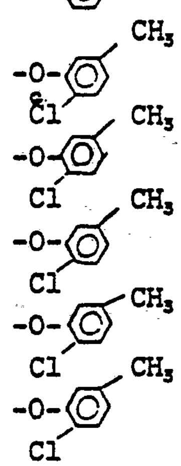 |
| $C_2H_5$ | 0 | |
| $C_4H_9$ | 0 | |
| 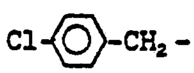 | 0 | |
| 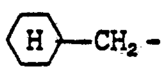 | 0 | |
| H | 1 | $-OC_4H_9$ |
| 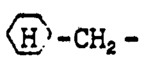 | 1 | $-OC_4H_9$ |
| $C_2H_5$ | 1 | $-O-C_4H_9$ |
| $C_4H_9$ | 1 | $-O-C_4H_9$ |
| 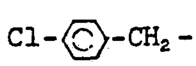 | 1 | $-O-C_4H_9$ |
| 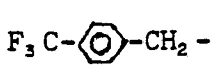 | 0 | $-COOC_2H_5$ |
| 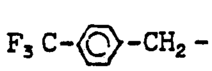 | 0 | $-COOC_3H_7$-i |
| 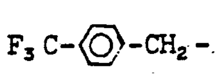 | 0 | $-CON(CH_3)_2$ |
| 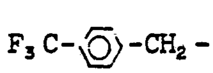 | 0 | 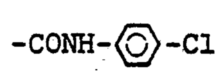 |

(Fortsetzung)

| $R^1$ | n | $R^2$ |
|---|---|---|
| 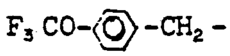 | 0 | $-COOCH_3$ |
| 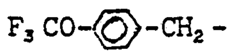 | 0 | -CN |
| H | 0 | -CN |
| H | 0 | 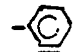 |
| H | 0 | 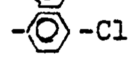 |
| H | 0 | 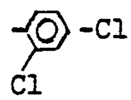 |
| H | 0 | 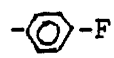 |
| H | 1 | 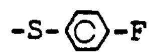 |
| $-C_4H_9-n$ | 1 | 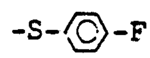 |
| 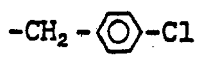 | 1 | 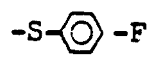 |
| 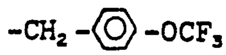 | 1 | 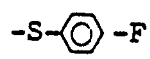 |
| 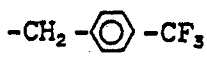 | 1 | 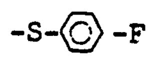 |
| H | 1 | 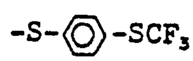 |
| $-C_4H_9-n$ | 1 | 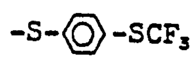 |
| 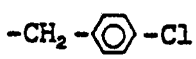 | 1 | 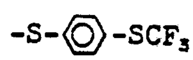 |
| 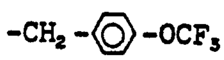 | 1 | 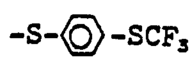 |
| 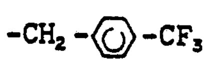 | 1 | 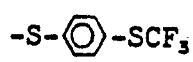 |
| H | 1 | 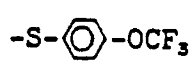 |
| $-C_4H_9-n$ | 1 | 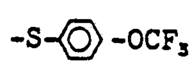 |
| 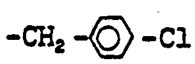 | 1 | 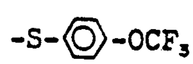 |
| 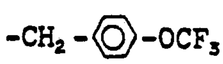 | 1 | 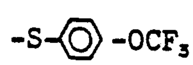 |
| 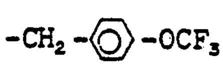 | 1 | 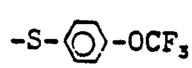 |

(Fortsetzung)

| $R^1$ | n | $R^2$ |
|---|---|---|
| H | 1 | 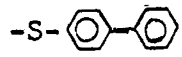 |
| $-C_4H_9-n$ | 1 | 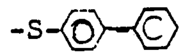 |
| 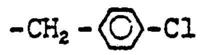 | 1 | 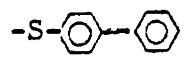 |
| 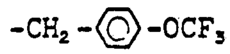 | 1 | 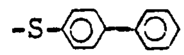 |
| 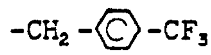 | 1 | 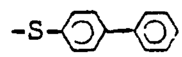 |
| H | 1 | 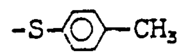 |
| $-C_4H_9-n$ | 1 | 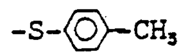 |
| 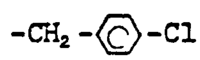 | 1 | 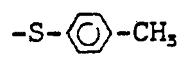 |
| 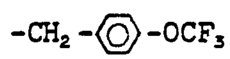 | 1 | 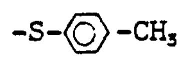 |
| 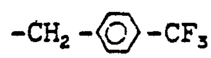 | 1 | 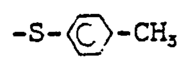 |
| H | 1 | 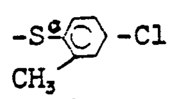 |
| $-C_4H_9-n$ | 1 | 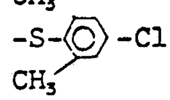 |
| 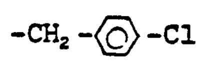 | 1 | 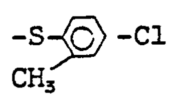 |
| 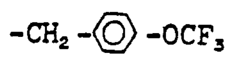 | 1 | 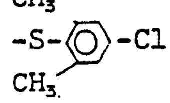 |
| 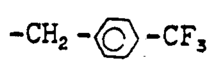 | 1 | 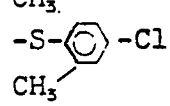 |
| H | 1 | 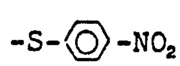 |
| $-C_4H_9-n$ | 1 | 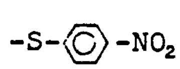 |
| 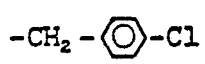 | 1 | 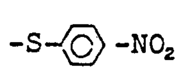 |
| 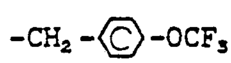 | 1 | 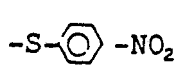 |
| 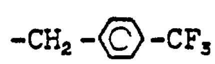 | 1 | 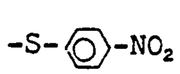 |

(Fortsetzung)

| $R^1$ | n | $R^2$ |
|---|---|---|
| H | 1 | 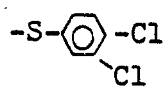 |
| $-C_4H_9$-n | 1 | 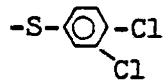 |
| 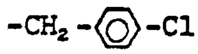 | 1 | 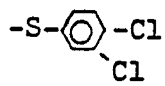 |
| 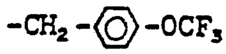 | 1 | 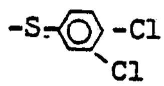 |
| 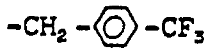 | 1 | 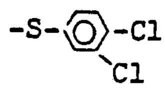 |
| H | 1 | 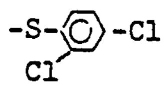 |
| $-C_4H_9$-n | 1 | 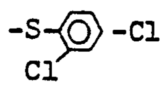 |
| 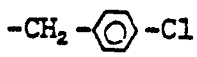 | 1 | 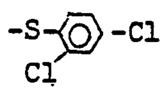 |
| 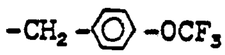 | 1 | 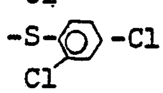 |
| 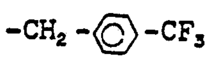 | 1 | 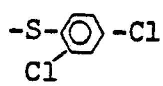 |
| H | 1 | 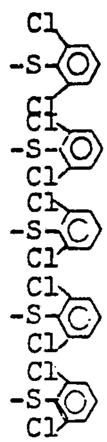 |
| $-C_4H_9$-n | 1 | |
| 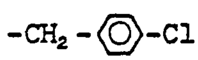 | 1 | |
| 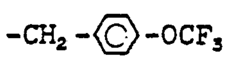 | 1 | |
| 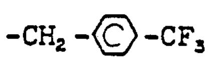 | 1 | |
| H | 1 | 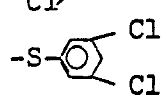 |
| $-C_4H_9$-n | 1 | 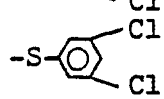 |
| 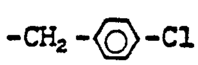 | 1 | 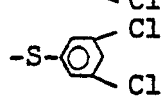 |
| 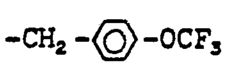 | 1 | 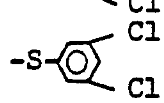 |
| 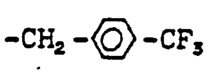 | 1 | 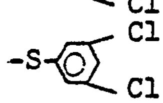 |

(Fortsetzung)

| $R^1$ | n | $R^2$ |
|---|---|---|
| H | 1 | $-S-C_4H_9-n$ |
| $-C_4H_9-n$ | 1 | $-S-C_4H_9-n$ |
| $-CH_2-$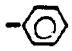$-Cl$ | 1 | $-S-C_4H_9-n$ |
| $-CH_2-$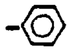$-OCF_3$ | 1 | $-S-C_4H_9-n$ |
| $-CH_2-$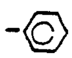$-CF_3$ | 1 | $-S-C_4H_9-n$ |
| H | 1 | $-SCH_3$ |
| $-C_4H_9-n$ | 1 | $-SCH_3$ |
| $-CH_2-$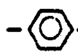$-Cl$ | 1 | $-SCH_3$ |
| $-CH_2-$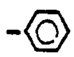$-OCF_3$ | 1 | $-SCH_3$ |
| $-CH_2-$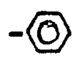$-CF_3$ | 1 | $-SCH_3$ |
| H | 1 | $-S-CH_2-$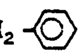 |
| $-C_4H_9-n$ | 1 | $-S-CH_2-$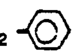 |
| $-CH_2-$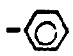$-Cl$ | 1 | $-S-CH_2-$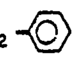 |
| $-CH_2-$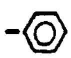$-OCF_3$ | 1 | $-S-CH_2-$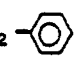 |
| $-CH_2-$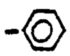$-CF_3$ | 1 | $-S-CH_2-$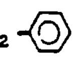 |
| H | 1 | $-S-CH_2-$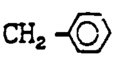$-Cl$ |
| $-C_4H_9-n$ | 1 | $-S-CH_2-$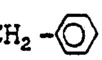$-Cl$ |
| $-CH_2-$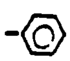$-Cl$ | 1 | $-S-CH_2-$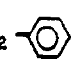$-Cl$ |
| $-CH_2-$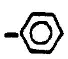$-OCF_3$ | 1 | $-S-CH_2-$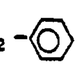$-Cl$ |
| $-CH_2-$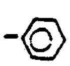$-CF_3$ | 1 | $-S-CH_2-$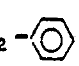$-Cl$ |
| H | 1 | $-S-CH_2-$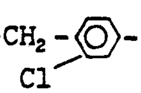$-Cl$ |

(Fortsetzung)

| R[1] | n | R[2] |
|---|---|---|
| $-C_4H_9-n$ | 1 | 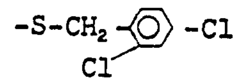 |
| 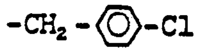 | 1 | 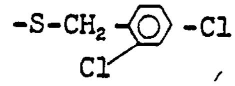 |
| 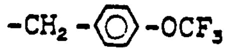 | 1 | 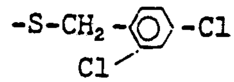 |
| 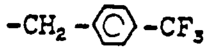 | 1 | 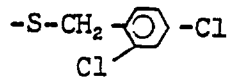 |
| H | 1 | 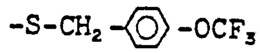 |
| $-C_4H_9-n$ | 1 | 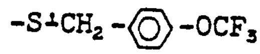 |
| 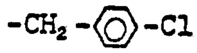 | 1 | 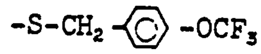 |
| 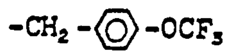 | 1 | 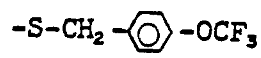 |
| 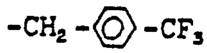 | 1 | 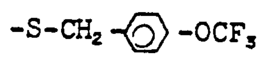 |
| H | 1 | 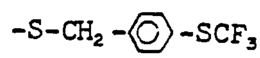 |
| $-C_4H_9-n$ | 1 | 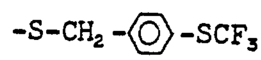 |
| 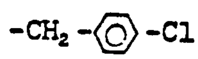 | 1 | 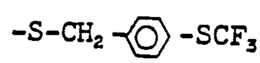 |
| 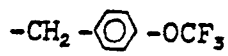 | 1 | 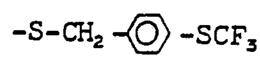 |
| 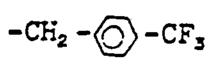 | 1 | 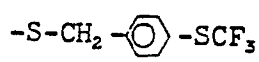 |
| H | 1 | 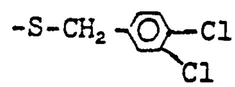 |
| $-C_4H_9-n$ | 1 | 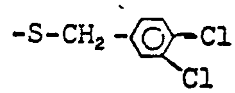 |
| 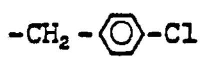 | 1 | 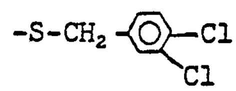 |
| 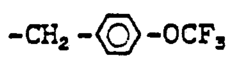 | 1 | 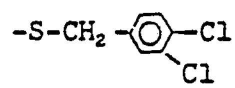 |
| 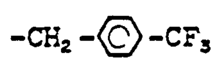 | 1 | 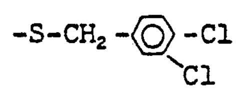 |
| H | 1 | 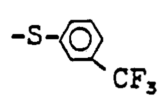 |

(Fortsetzung)

| $R^1$ | n | $R^2$ |
|---|---|---|
| $-C_4H_9-n$ | 1 | 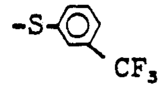 |
| 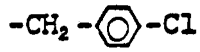 | 1 | 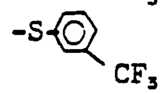 |
| 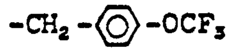 | 1 | 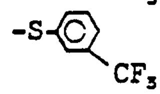 |
| 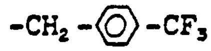 | 1 | 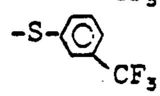 |
| H | 1 | 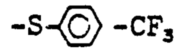 |
| $-C_4H_9-n$ | 1 | 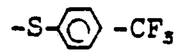 |
| 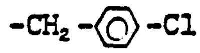 | 1 | 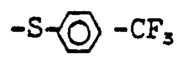 |
| 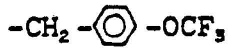 | 1 | 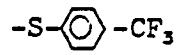 |
| 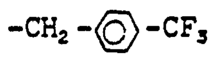 | 1 | 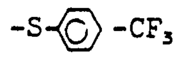 |
| H | 1 | 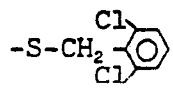 |
| $-C_4H_9-n$ | 1 | 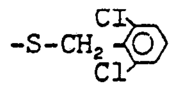 |
| 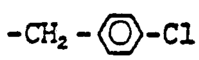 | 1 | 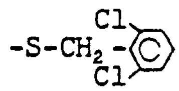 |
| 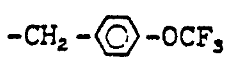 | 1 | 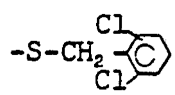 |
| 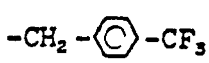 | 1 | 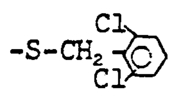 |
| H | 1 | 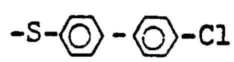 |
| $-C_4H_9-n$ | 1 | 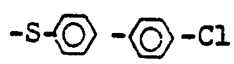 |
| 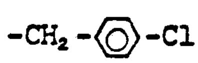 | 1 | 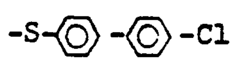 |
| 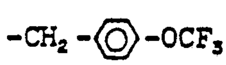 | 1 | 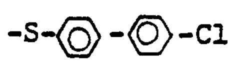 |
| 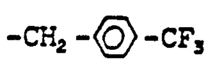 | 1 | 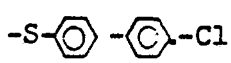 |
| H | 1 | 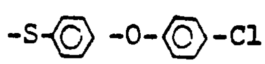 |
| $-C_4H_9-n$ | 1 | 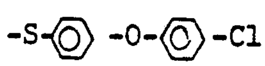 |

(Fortsetzung)

| $R^1$ | n | $R^2$ |
|---|---|---|
| 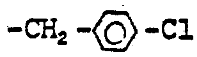 | 1 | 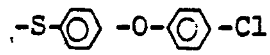 |
| 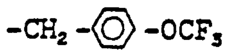 | 1 | 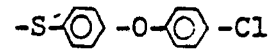 |
| 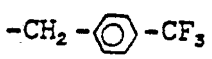 | 1 | 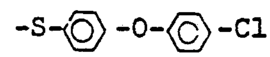 |
| H | 1 | 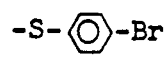 |
| $-C_4H_9$-n | 1 | 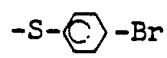 |
| 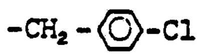 | 1 | 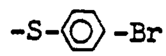 |
| 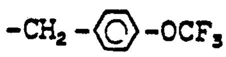 | 1 | 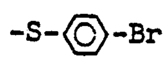 |
| 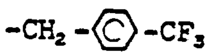 | 1 | 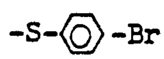 |
| H | 1 | 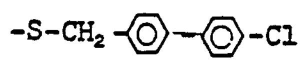 |
| $-C_4H_9$-n | 1 | 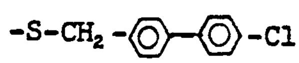 |
| 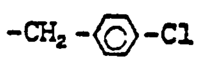 | 1 | 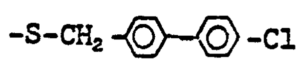 |
| 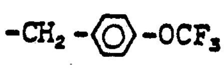 | 1 | 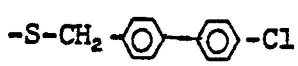 |
| 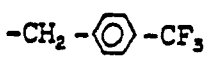 | 1 | 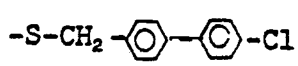 |
| H | 1 | 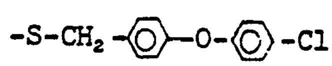 |
| $-C_4H_9$-n | 1 | 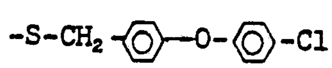 |
| 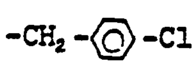 | 1 | 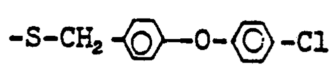 |
| 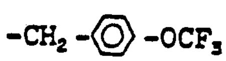 | 1 | 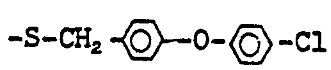 |
| 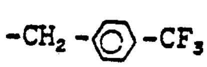 | 1 | 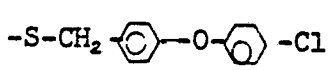 |
| H | 1 | 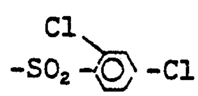 |
| $-C_4H_9$-n | 1 | 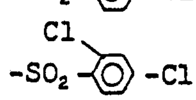 |
| 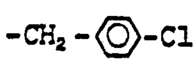 | 1 | 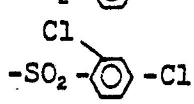 |

(Fortsetzung)

| R¹ | n | R² |
|---|---|---|
| 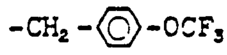 | 1 | 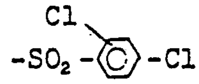 |
| 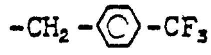 | 1 | 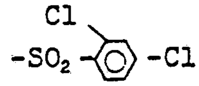 |
| H | 1 | 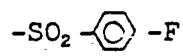 |
| $-C_4H_9$-n | 1 | 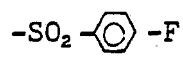 |
| 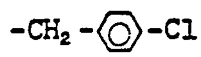 | 1 | 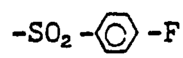 |
| 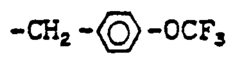 | 1 | 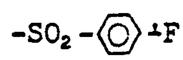 |
| 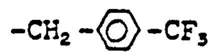 | 1 | 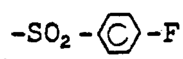 |
| H | 1 | 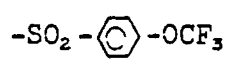 |
| $-C_4H_9$-n | 1 | 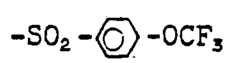 |
| 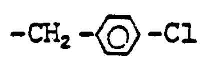 | 1 | 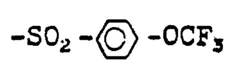 |
| 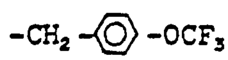 | 1 | 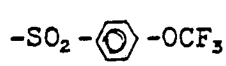 |
| 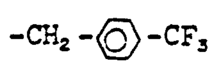 | 1 | 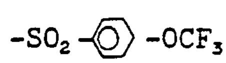 |
| H | 1 | 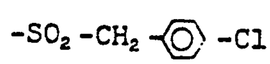 |
| $-C_4H_9$-n | 1 | 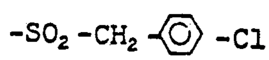 |
| 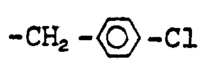 | 1 | 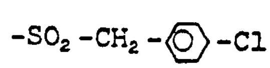 |
| 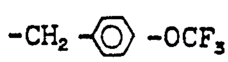 | 1 | 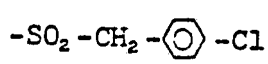 |
| 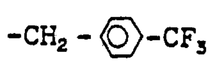 | 1 | 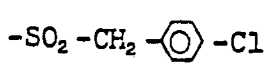 |

Verwendet man beispielsweise 5-(4-Chlorphenyl)-2-(2,4-dichlorphenoxy)-2-methyl-4-(1,2,4-triazol-1-yl)-pentan-3-on und Natriumborhydrid als Ausgangsstoffe, so kann der Reaktionsablauf durch das folgende Formelschema wiedergegeben werden :

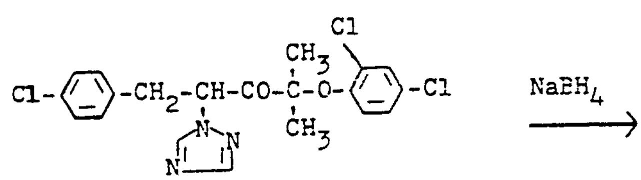

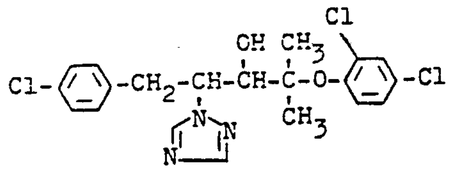

(III)

Die für das erfindungsgemäße Verfahren als Ausgangsstoffe zu verwendenden substituierten 1-Azolyl-butan-2-one sind durch die Formel II allgemein definiert. In dieser allgemeinen Formel stehen Az, $R^1$, $R^2$ und der Index n vorzugsweise für diejenigen Bedeutungen, die bei den erfindungsgemäßen Verbindungen der allgemeinen Formel I bereits vorzugsweise für diese Substituenten genannt wurden.

Die substituierten 1-Azolyl-butan-2-one der allgemeinen Formel II sind noch nicht bekannt ; sie sind teilweise Gegenstand einer eigenen älteren Anmeldung, die noch nicht veröffentlicht ist (vergleiche deutsche Patentanmeldung P 3028330 vom 25.7.1980), bzw. einer eigenen parallelen Patentanmeldung und werden erhalten, indem man

a) Halogenketone der allgemeinen Formel III

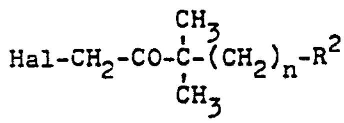

(III)

in welcher

Hal für Halogen, insbesondere Chlor oder Brom steht und

$R^2$ und n die oben angegebene Bedeutung haben, wobei jedoch in der Gruppierung —X—$R^3$ der Substituent X nur für Sauerstoff oder Schwefel steht,

mit Azolen der allgemeinen Formel IV

H—Az (IV)

in welcher

Az die oben angegebene Bedeutung hat,

in Gegenwart eines inerten organischen Lösungsmittels, wie beispielsweise Acetonitril, und in Gegenwart eines Säurebindemittels, wie beispielsweise Kaliumcarbonat oder ein Überschuß an Azol, bei Temperaturen zwischen 60 und 120 °C umsetzt ; und gegebenenfalls

b) die so erhaltenen Verbindungen der allgemeinen Formel II

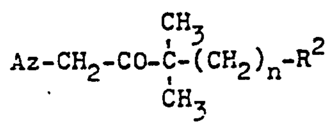

(IIa)

in welcher

Az, n und $R^2$ die oben angegebene Bedeutung haben,

mit einem Alkylierungsmittel der allgemeinen Formel V

$R^1$—Z (V)

in welcher

$R^1$ die oben angegebene Bedeutung hat und

Z für eine elektronenanziehende Abgangsgruppe, wie Halogen, p-Methylphenylsulfonyloxy oder Sulfat steht,

in üblicher Weise in Gegenwart eines inerten organischen Lösungsmittels, wie beispielsweise Dimethylsulfoxid, bei Temperaturen zwischen 0 und 100 °C umsetzt und gegebenenfalls

c) die nach den Verfahren (a) und (b) erhaltenen Verbindungen der allgemeinen Formel IIb

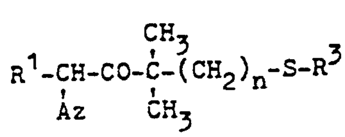

(IIb)

in welcher

Az, $R^1$, n und $R^3$ die oben angegebene Bedeutung haben,

nach bekannten Methoden in üblicher Weise oxidiert, wie z. B. durch Umsetzung mit Wasserstoffperoxid in Eisessig bei Temperaturen zwischen – 30 und 80 °C. Bei der Durchführung der Oxidation setzt man auf 1 Mol der Verbindungen der allgemeinen Formel IIb etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen – 30 bis + 30 °C, entstehen vorzugsweise diejenigen Verbindungen der allgemeinen Formel II, bei denen X für SO steht. Bei Überschuß an Oxidationsmitteln und höheren Temperaturen (10 bis 80 °C) entstehen vorzugsweise solche Verbindungen der allgemeinen Formel II, bei denen X für $SO_2$ steht. Die Isolierung der Oxidationsprodukte erfolgt in üblicher Weise.

Die Halogenketone der allgemeinen Formel III sind teilweise bekannt (vergleiche DE-OS 2 635 663, teilweise sind sie Gegenstand eigener älterer Anmeldungen, die noch nicht veröffentlicht sind (vergleiche z. B. deutsche Patentanmeldung P3021551 vom 7.6.1980), und teilweise sind sie völlig neu. Sie werden erhalten, indem man Ketone der allgemeinen Formel VI

$$CH_3-CO-\underset{CH_3}{\overset{CH_3}{C}}-(CH_2)_n-R^2 \qquad \text{(VI)}$$

in welcher

$R^2$ und n die oben angegebene Bedeutung haben,

mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei 20 bis 60 °C umsetzt.

Die Ketone der allgemeinen Formel VI sind teilweise bekannt [vergleiche J. Org. Chem. 42, 1709-1717 (1977) ; J. Am. Chem. Soc. 98, 7882-84 (1976) ; J. Org. Chem. 37, 2834-2840 (1972), US-Patentschrift 3 937 738 sowie C.A. 82, 30898 j (1975)] ; teilweise sind sie Gegenstand eigener älterer Anmeldungen, die noch nicht veröffentlicht sind (vergleiche z. B. deutsche Patentanmeldung P 30 21 516 vom 7.6.1980 ; teilweise sind sie neu. Sie können nach den dort angegebenen Verfahren erhalten werden, indem man z. B. Keto-Derivate der allgemeinen Formel VII

$$CH_3-CO-\underset{CH_3}{\overset{CH_3}{C}}-(CH_2)_n-Y \qquad \text{(VII)}$$

in welcher

n die oben angegebene Bedeutung hat und

Y für Chlor, Brom oder die Gruppierung $-O-SO_2R^6$ steht, wobei

$R^6$ für Alkyl mit 1 bis 4 Kohlenstoffatomen sowie für gegebenenfalls durch Alkyl mit 1 bis 4 Kohlenstoffatomen substituiertes Phenyl steht,

mit Verbindungen der allgemeinen Formel VIII

$$Me-R^2 \qquad \text{(VIII)}$$

in welcher

$R^2$ die oben angegebene Bedeutung hat und

Me für ein Alkalimetall, wie vorzugsweise Natrium und Kalium, oder Wasserstoff steht,

in Gegenwart eines organischen Lösungsmittels, wie beispielsweise Glykol oder Dimethylformamid, und gegebenenfalls in Gegenwart eines Säurebindemittels, wie beispielsweise Natriumcarbonat, bei Temperaturen zwischen 80 und 150 °C umsetzt.

Die Keto-Derivate der allgemeinen Formel VII sind bekannt [vergleiche z. B. DE-OS 2 632 603 und J. Org. Chem. 35, 2391 (1970)], bzw. können sie in allgemein bekannter Art und Weise erhaltenwerden.

Die Verbindungen der allgemeinen Formel VIII sind allgemein bekannte Verbindungen der organischen Chemie und werden gegebenenfalls *in situ* eingesetzt.

Die Azole der allgemeinen Formel IV und die Alkylierungsmittel der allgemeinen Formel V sind ebenfalls allgemein bekannte Verbindungen der organischen Chemie.

Die erfingungsgemäße Reduktion erfolgt in üblicher Weise, wie z. B. durch Umsetzung mit komplexen Hydriden, gegebenenfalls in Gegenwart eines Verdünnungsmittels ; oder durch Umsetzung mit Aluminiumisopropylat in Gegenwart eines Verdünnungsmittels.

Arbeitet man mit komplexen Hydriden, so kommen als Verdünnungsmittel für die erfingungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran.

Die Reaktion wird in allgemeinen bei 0 bis 30 °C, vorzugsweise bei 0 bis 20 °C durchgeführt. Hierzu

setzt man auf 1 Mol des Ketones der allgemeinen Formel II etwa 1 Mol eines komplexen Hydrids, wie Natriumhydrid oder Lithiumalanat, ein. Zur Isolierung der reduzierten Verbindungen der allgemeinen Formel I wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der allgemeinen Formel II etwa 1 bis 2 Mol Aluminiumisopropylat ein. Zur Isolierung der reduzierten Verbindungen der allgemeinen Formel I wird das überschüssige Lösungsmittel durch Destillation in Vakuum entfernt und die entstandene Aluminiumverbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der allgemeinen Formel I kommen vorzugsweise folgende Säuren infrage ; die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionnelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der allgemeinen Formel I können in einfacher Weise nach üblichen Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z. B. durch Lösen des Metallsalzes in Alkohol, z. B. Ethanol und Hinzufügen zur Verbindung der allgemeinen Formel I. Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chrytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrugen, so zur Bekämpfung von Erysiphe-Arten, wie z. B. gegen den Erreger des Gersten- bzw. des Getreidemehltaus (Erysiphe graminis) oder des Gurkenmehltaus (Erysiphe cichoracearum). Hervorzuheben ist die teilweise systemische Wirkung der erfindungsgemäßen Stoffe. So gelingt es, Pflanzen gegen Pilzbefall zu schützen, wenn man den Wirkstoff über den Boden und die Wurzel den oberirdischen Teilen der Pflanze zuführt.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte

verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakandau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jaho zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schießlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Sticktoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

Bei der Verwendung als Pflanzenwachstumsregulatoren können die Wirkstoffkonzentrationen in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

In entsprechenden höheren Aufwandmengen zeigen die erfindungsgemäßen Verbindungen auch eine herbizide Wirkung.

## Herstellungsbeispiele

### Beispiel 1

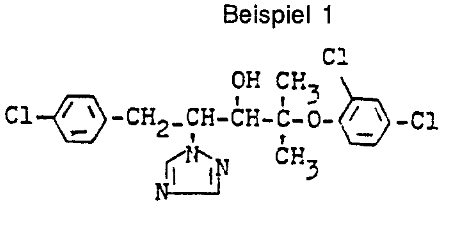

x HCl

10 g (0,022 8 Mol) 5-(4-Chlorphenyl)-2-(2,4-dichlorphenoxy)-2-methyl-4-(1,2,4-triazol-1-yl)-pentan-3-on werden in 100 ml Methanol gelöst, 1 g Natriumborhydrid portionsweise bei 0-5 °C zugegeben, 15 Stunden bei Raumtemperatur nachgerührt und 50 ml 2n Salzsäure zugegeben. Nach 4 Stunden wird durch Abdestillieren des Lösungsmittels im Vakuum eingeengt. Der Rückstand wird in 200 ml Methylenchlorid aufgenommen, die organische Phase mit 100 ml gesättigter Natriumhydrogencarbonatlösung verrührt, die organische Phase abgetrennt, zweimal mit je 100 ml Wasser gewaschen, über Natriumsulfat getrocknet und im Vakuum eingeengt. Der Rückstand wird in 100 ml Ether gelöst und mit etherischer Salzsäure versetzt. Man erhält 7,8 g (81 % Theorie) 5-(4-Chlorphenyl)-2-(2,4-dichlorphenoxy)-2-methyl-4-(1,2,4-triazol-1-yl)-pentan-3-ol-hydrochlorid vom Schmelzpunkt 46-50 °C.

Herstellung des Ausgangsproduktes

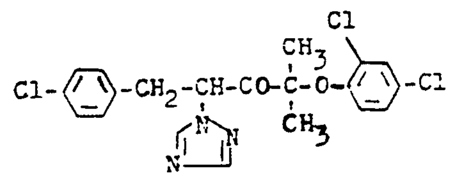

Zu 31,4 g (0,1 Mol) 3-(2,4-Dichlorphenoxy)-3-methyl-1-(1,2,4-triazol-1-yl)-butan-2-on in 100 ml Dimethylsulfoxid werden unter Kühlung bei 20 °C zunächst 5,6 g Kaliumhydroxid in 12 ml Wasser und danach 16,1 g (0,1 Mol) 4-Chlorbenzylchlorid in 5 ml Dimethylsulfoxid getropft. Man läßt 15 Stunden bei 20 °C nachreagieren, gibt die Lösung auf 200 ml Wasser, extrahiert mit 200 ml Methylenchlorid, wäscht die organische Phase dreimal mit je 200 ml Wasser, trocknet über Natriumsulfat und destilliert das Lösungsmittel im Wasserstrahlvakuum ab. Der Rückstand wird in 200 ml Ether aufgenommen, unter Rückfluß erhitzt und die ausgefallenen Kristalle abgesaugt. Man erhält 21,3 g (48 % der Theorie) 5-(4-Chlorphenyl)-2-(2,4-dichlorphenoxy)-2-methyl-4-(1,2,4-triazol-1-yl)-pentan-3-on vom Schmelzpunkt 104-108 °C.

Beispiel 2

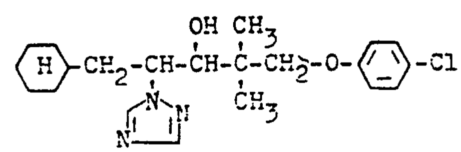

10 g (0,034 Mol) 1-(4-Chlorphenoxy)-5-cyclohexyl-2,2-dimethyl-4-(1,2,4-triazol-1-yl)-pentan-3-on werden in 100 ml Methanol gelöst, 1,7 g Natriumborhydrid bei 0-5 °C portionsweise zugegeben, 15 Stunden bei Raumtemperatur nachgerührt und 20 ml 2n Salzsäure zugetropft. Nach 5 Stunden wird durch Abdestillieren des Lösungsmittels im Vakuum eingeengt, der Rückstand in 100 ml Methylenchlorid aufgenommen, die organische Phase mit 100 ml gesättigter Natriumhydrogencarbonatlösung verrührt, die organische Phase abgetrennt, zweimal mit je 50 ml Wasser gewaschen, über Natriumsulfat getrocknet und im Vakuum eingeengt. Der Rückstand wird mit 50 ml Diisopropylether verrieben. Man erhält 7,2 g (54 % der Theorie) 1-(4-Chlorphenoxy)-5-cyclohexyl-2,2-dimethyl-4-(1,2,4-triazol-1-yl)-pentan-3-ol vom Schmelzpunkt 105-108 °C.

Herstellung des Ausgangsproduktes

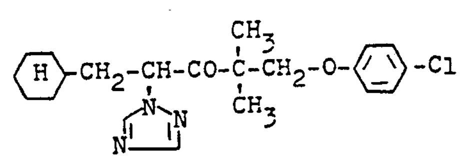

Zu 29,3 g (0,1 Mol) 1-(4-Chlorphenoxy)-2,2-dimethyl-4-(1,2,4-triazol-1-yl)-butan-3-on in 100 ml Dimethylsulfoxid werden bei 20 °C zunächst 5,6 g Kaliumhydroxid in 12 ml Wasser und danach 17,7 g (0,1 Mol) Cyclohexylmethylbromid in 5 ml Dimethylsulfoxid getropft. Man läßt das Reaktionsgemisch 15 Stunden bei Raumtemperatur nachrühren, gibt es auf 200 ml Wasser und extrahiert mit 200 ml Methylenchlorid. Die organische Phase wird dreimal mit je 100 ml Wasser gewaschen, über Natriumsulfat getrocknet und durch Abdestillieren des Lösungsmittels eingeengt. Der Rückstand wird in 100 ml Ether aufgenommen, wobei er auskristallisiert. Man erhält 18,6 g (47 % der Theorie) 1-(4-Chlorphenoxy)-5-cyclohexyl-2,2-dimethyl-4-(1,2,4-triazol-1-yl)-pentan-3-on vom Schmelzpunkt 58-60 °C.

Beispiel 3

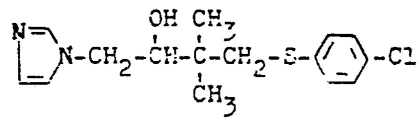

0,3 g (0,007 9 Mol) Natriumborhydrid in 8 ml Wasser werden bei Raumtemperatur zu 7 g (0,024 Mol) 4-(4-Chlorphenylmercapto)-3,3-dimethyl-1-(imidazol-1-yl)-butan-2-on getropft. Man läßt 1 Stunde bei Raumtemperatur nachrühren und gießt das Reaktionsgemisch auf Wasser. Die ausgefallenen Kristalle werden abgesaugt und bei 50 °C im Vakuum getrocknet. Man erhält 6,5 g (91 % der Theorie) 4-(4-Chlorphenylmercapto)-3,3-dimethyl-1-(imidazol-1-yl)-butan-2-ol vom Schmelzpunkt 109-110 °C.

Herstellung des Ausgangsproduktes

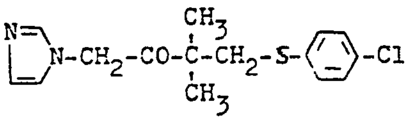

199 g (0,618 Mol) 1-Brom-4-(4-chlorphenylmercapto)-3,3-dimethyl-butan-2-on, 120 g (1,76 Mol) Imidazol und 243,5 g (1,76 Mol) Kaliumcarbonat in 3 l Aceton werden 5 Stunden unter Rückfluß gerührt. Danach läßt man abkühlen, saugt die anorganischen Salze ab und engt das Filtrat ein. Der Rückstand wird in Methylenchlorid aufgenommen, dreimal mit Wasser gewaschen, über Natriumsulfat getrocknet und mit Wasser gewaschen, über Natriumsulfat getrocknet und eingeengt. Nach Umkristallisation aus Diisopropylether erhält man 156 g (82 % der Theorie) 4-(4-Chlorphenylmercapto)-3,3-dimethyl-1-(imidazol-1-yl)-butan-2-on vom Schmelzpunkt 50 °C.

In entsprechender Weise werden die folgenden erfindungsgemäßen Verbindungen der allgemeinen Formel I

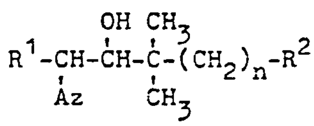 (I)

erhalten :

| Beispiel Nr. | $R^1$ | Az | n | $R^2$ | Schmelzpunkt (°C) Brechungsindex $n_D^{20}$; Siedepunkt (°C)/Torr |
|---|---|---|---|---|---|
| 4 | H | 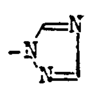 | 1 | 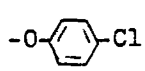 | zähes Öl |
| 5 | H | 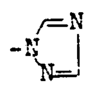 | 1 | 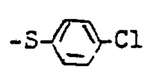 | zähes Öl |
| 6 | H | 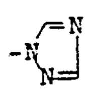 | 1 | 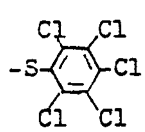 | 148 |
| 7 | 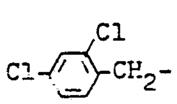 | 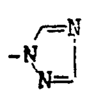 | 1 | $-O-CH_3$ | 120(Zers.)(x HCl) |
| 8 | 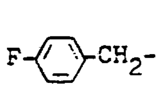 | 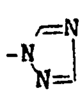 | 1 | $-O-CH_3$ | 86-90 |

(Fortsetzung)

| Beispiel Nr. | $R^1$ | Az | n | $R^2$ | Schmelzpunkt (°C); Brechungsindex $n_D^{20}$; Siedepunkt (°C)/Torr |
|---|---|---|---|---|---|
| 9 | 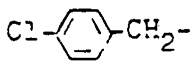 | 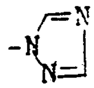 | 1 | $-O-CH_3$ | 104-108 |
| 10 | 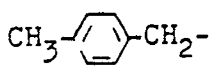 | 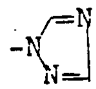 | 1 | 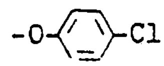 | 158 |
| 11 | 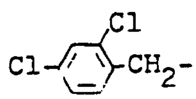 | 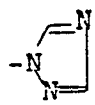 | 1 | 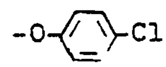 | 128 |
| 12 | 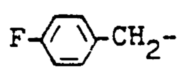 | 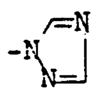 | 1 | 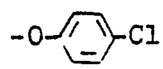 | 167-168 |
| 13 | $C_4H_9$-n | 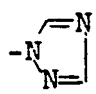 | 1 | 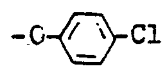 | zähes Öl |
| 14 | H | 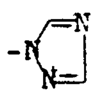 | 0 | 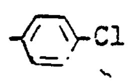 | 109 |
| 15 | H | 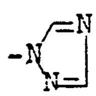 | 0 | 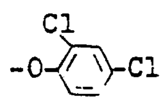 | 89 |
| 16 | H | 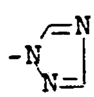 | 0 | 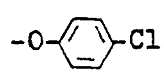 | 121-123 |
| 17 | 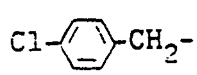 | 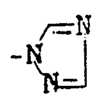 | 0 | $-CO-OC_2H_5$ | 120 |
| 18 | H | 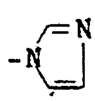 | 1 | 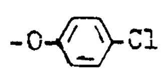 | 96-98 |
| 19 | H | 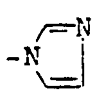 | 1 | 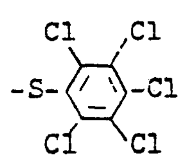 | 154-156 |
| 20 | 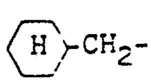 | 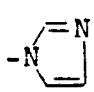 | 1 | 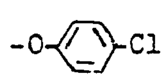 | 67-70 |
| 21 | H | 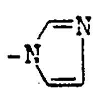 | 0 | 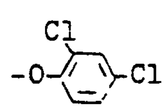 | 120-124 |

(Fortsetzung)

| Beispiel Nr. | $R^1$ | Az | n | $R^2$ | Schmelzpunkt (°C); Brechungsindex $n_D^{20}$; Siedepunkt (°C)/Torr |
|---|---|---|---|---|---|
| 22 | H | 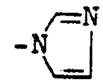 | 0 | 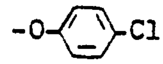 | 114-12o |
| 23 | H | 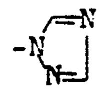 | 1 | 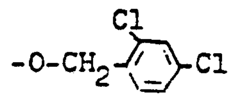 | 125-127 |
| 24 | 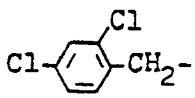 | 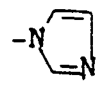 | 1 | 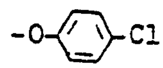 | 164-168 |
| 25 | 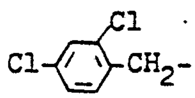 | 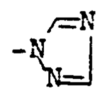 | 0 | 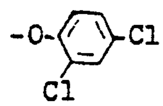 | 192-194 (x 1/2 NDS) |
| 26 | 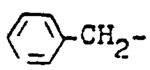 | 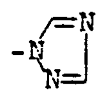 | 0 | 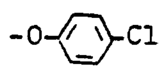 | 178-18o (x 1/2 NDS) |
| 27 | 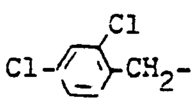 | 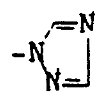 | 0 | 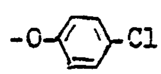 | 45-48 |
| 28 | 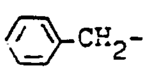 | 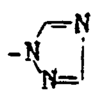 | 0 | 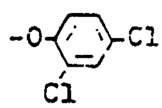 | 2o4 (x 1/2 NDS) |
| 29 | 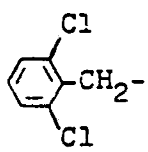 | 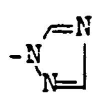 | 0 | 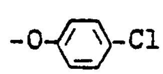 | 15o (x 1/2 NDS) |
| 3o | 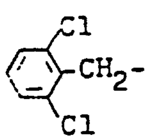 | 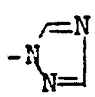 | 0 | 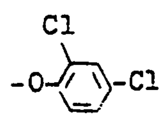 | 2oo (x 1/2 NDS) |
| 31 | 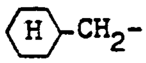 | 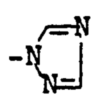 | 0 | 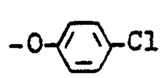 | 124 |
| 32 | 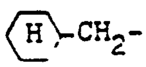 | 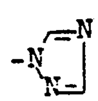 | 0 | 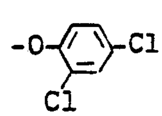 | 176-178 (x 1/2 NDS) |
| 33 | H | 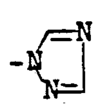 | 1 | $-OC_2H_5$ | 1,4671 |

NDS = 1,5-Naphthalindisulfonsäure)

(Fortsetzung)

| Bei-spiel Nr. | $R^1$ | Az | n | $R^2$ | Schmelzpunkt (°C); Brechungsindex $n_D^{20}$; Siedepunkt (°C)/Torr |
|---|---|---|---|---|---|
| 34 | 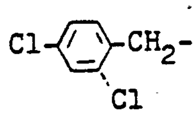 | 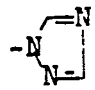 | 1 | 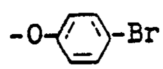 | 140-142 |
| 35 | 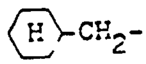 | 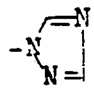 | 1 | 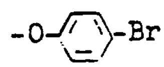 | 148 |
| 36 | 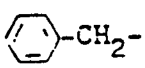 | 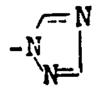 | 1 | 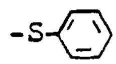 | 78-80 |
| 37 | 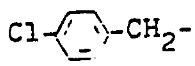 | 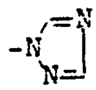 | 1 | 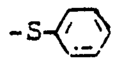 | 25 |
| 38 | 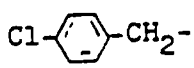 | 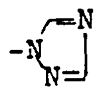 | 1 | 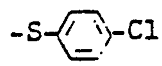 | 124-126 |
| 39 | 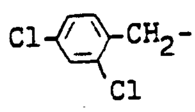 | 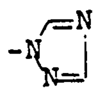 | 1 | 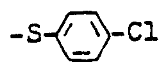 | 48 |
| 40 | 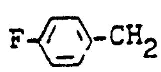 | 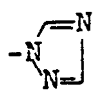 | 1 | 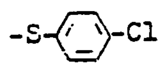 | 152-153 |
| 41 | $C_4H_9$-n | 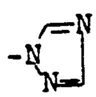 | 1 | 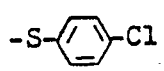 | 1,5458 |
| 42 | 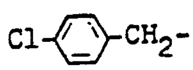 | 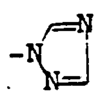 | 1 | $-OC_2H_5$ | 40 |
| 43 | H | 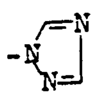 | 1 | 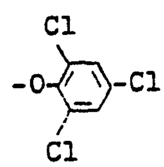 | 1,5279 |
| 44 | H | 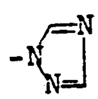 | 1 | 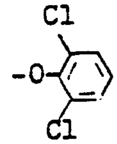 | 130-135 |
| 45 | 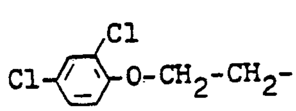 | 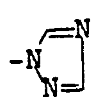 | 1 | 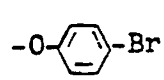 | 69-76 |
| 46 | $C_4H_9$ -n | 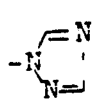 | 1 | 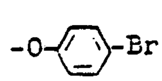 | 69-72 |
| 47 | 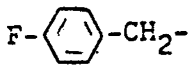 | 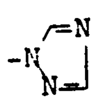 | 1 | 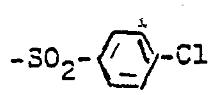 | 40 |

(Fortsetzung)

| Beispiel Nr. | $R^1$ | Az | n | $R^2$ | Schmelzpunkt (°C) Brechungsindex $n_D^{20}$; Siedepunkt (°C)/Torr |
|---|---|---|---|---|---|
| 48 | 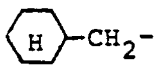 | 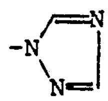 | 1 | $-OC_2H_5$ | 30 |
| 49 | 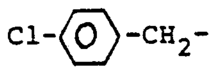 | 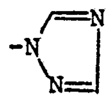 | 1 | 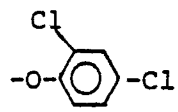 | 145-48 |
| 50 | 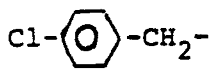 | 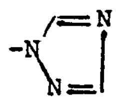 | 1 | 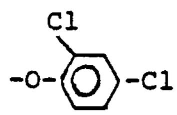 | 148 |
| 51 | $n-C_4H_9$ | " | 0 | 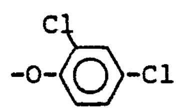 | 1,545 |
| 52 | " | " | 0 | 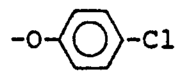 | 1,535 |
| 53 | $CH_2=CH-CH_2-$ | " | 0 | 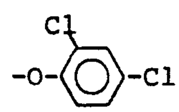 | 1,556 |
| 54 | $C_2H_5$ | " | 0 | " | 110 |
| 55 | $CH_3$ | " | 0 | " | 145 |
| 56 | $CH_2=CH-CH_2-$ | 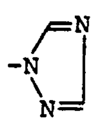 | 0 | 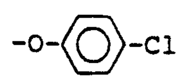 | 1,547 |
| 57 | $C_2H_5$ | " | 0 | " | 1,540 |
| 58 | $CH_3$ | " | 0 | " | 1,559 |
| 59 | $C_2H_5$ | " | 0 | 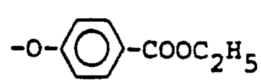 | 1,535 |
| 60 | $CH_2=CH-CH_2-$ | " | 0 | " | 1,521 |
| 61 | $CH_3$ | " | 0 | " | 84-96 |

(Fortsetzung)

| Beispiel Nr. | $R^1$ | Az | n | $R^2$ | Schmelzpunkt (°C) Brechungsindex $n_D^{20}$; Siedepunkt (°C)/Torr |
|---|---|---|---|---|---|
| 62 | n-$C_4H_9$- | " | O | " | 1,535 |
| 63 | 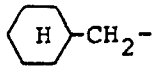 | " | O | " | 1,534 |
| 64 | 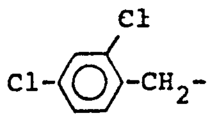 | " | O | " | 104-09 |
| 65 | $CH_2$=CH-$CH_2$- | 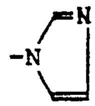 | O | 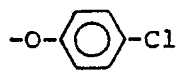 | 141-45 |
| 66 | $CH_2$=CH-$CH_2$- | " | O | 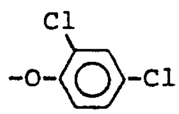 | 100-04 |
| 67 | 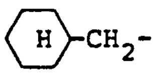 | " | O | 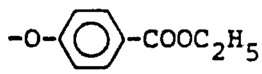 | 136-40 |
| 68 | $CH_2$=CH-$CH_2$- | " | O | " | 106-10 |
| 69 | 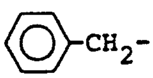 | " | O | " | 130-34 |
| 70 | H | 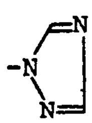 | 1 | 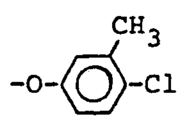 | 1,5218 |
| 71 | 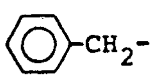 | " | 1 | 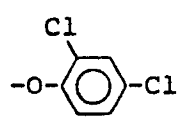 | 155-56 |
| 72 | 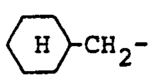 | " | 1 | " | 145-48 |
| 73 | H | " | 1 | 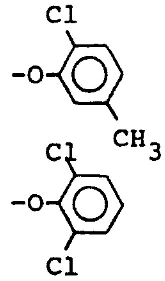 | 145 |
| 74 | 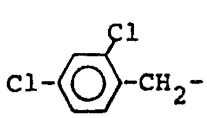 | " | 1 | | 114-20 |

(Fortsetzung)

| Beispiel Nr. | $R^1$ | Az | n | $R^2$ | Schmelzpunkt (°C) Brechungsindex $n_D^{20}$; Siedepunkt (°C)/Torr |
|---|---|---|---|---|---|
| 75 | H | " | 1 | 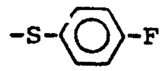 | 20 |
| 76 | H | 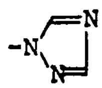 | 1 | 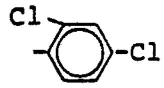 | 105 |
| 77 | H | " | 1 | 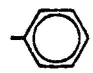 | 127 |
| 78 | H | 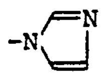 | 1 | 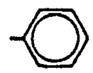 | 144 |
| 79 | 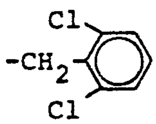 | 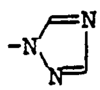 | 1 | 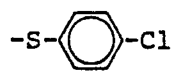 | 168 |
| 80 | 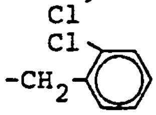 | " | 1 | 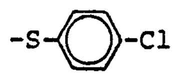 | ca. 30 |
| 81 | $-C_4H_9$-n | " | 1 | 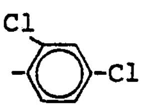 | 106 |
| 82 | $-C_4H_9$-n | " | 1 | 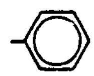 | 105 |
| 83 | 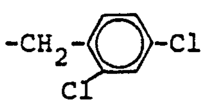 | " | 1 | 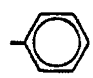 | 178 |
| 84 | $-C_4H_9$-n | " | 1 | 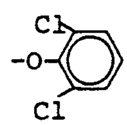 | 160-2 (x HCl) |
| 85 | 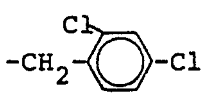 | 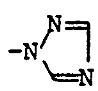 | 1 | $-O-C_2H_5$ | 155-9 (x HCl) |
| 86 | 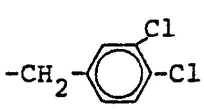 | " | 1 | $-O-C_2H_5$ | 148-50 (x HCl) |
| 87 | 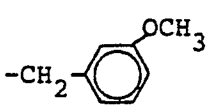 | " | 1 | 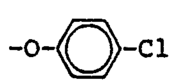 | 127-30 (x HCl) |
| 88 | 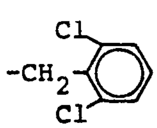 | 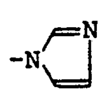 | 0 | 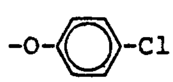 | 180-183 |
| 89 | $-C_4H_9$-n | " | 0 | 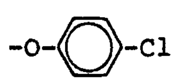 | 118-120 |

(Fortsetzung)

| Bsp. Nr. | $R^1$ | Az | n | $R^2$ | Schmelzpunkt (°C) Brechungsindex $n_D^{20}$; Siedepkt. (°C)/Torr |
|---|---|---|---|---|---|
| 90 | $-C_4H_9-n$ | 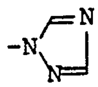 | 0 | 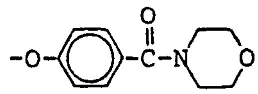 | Harz |
| 91 | $-CH_2-CH=CH_2$ | " | 0 | " | Harz |
| 92 | $-CH_2-$ 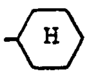 | " | 0 | " | Harz |
| 93 | $-CH_2-$ 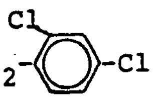 | " | 0 | " | 158-164 |
| 94 | $-CH_2-C\equiv CH$ | " | 1 | 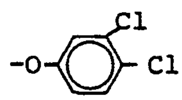 | 156-58 (x HCl) |
| 95 | $-CH_2-CH_2-CH=CH_2$ | " | 1 | " | 115-25 (x HCl) |
| 96 | H | 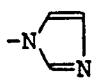 | 1 | 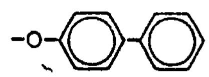 | 138-40 |
| 97 | H | 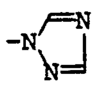 | 1 | " | 134-36 |
| 98 | $-CH_2-$ 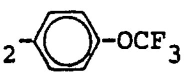 | " | 1 | 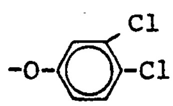 | 142-45 |
| 99 | $-C_2H_5$ | " | 1 | 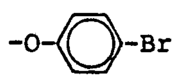 | 148-54 (x HCl) |
| 100 | $-C_4H_9(n)$ | 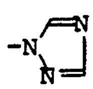 | 1 | 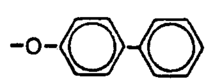 | 154-74 (xHCl) |
| 101 | $-C_4H_9(n)$ | 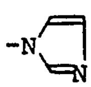 | 1 | " | Oel (xHCl) |
| 102 | $-C_2H_5$ | 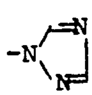 | 0 | 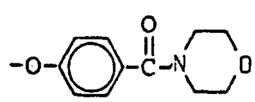 | 168-172 (x$\frac{1}{2}$ NDS) |
| 103 | $-C_2H_5$ | " | 0 | 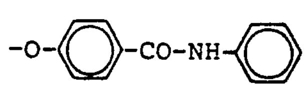 | 133-143 |
| 104 | $-C_2H_5$ | " | 0 | 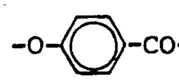 $-CO-N[CH_2CH(CH_3)_2]_2$ | 149-152 (x$\frac{1}{2}$ NDS) |

(Fortsetzung)

| Bsp. Nr. | $R^1$ | Az | n | $R^2$ | Schmelzpunkt (°C) Brechungsindex $n_D^{20}$; Siedepkt. (°C)/Torr |
|---|---|---|---|---|---|
| 105 | 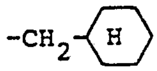 | " | 0 | 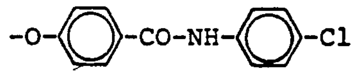 | 133-138 |
| 106 | " | " | 0 | 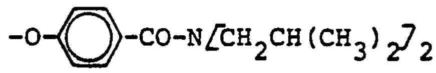 | Öl |
| 107 | 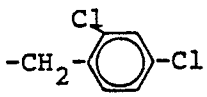 | " | 1 | 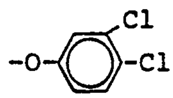 | 132-40 |
| 108 | $-C_4H_9$-n | " | 1 | " | 138-41 (x HCl) |
| 109 | 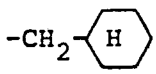 | " | 1 | 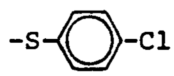 | $n_D^{20}$ = 1,5624 |
| 110 | $CH_2=CH-CH_2-$ | 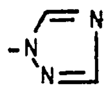 | 1 | 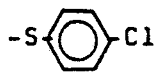 | 1,5739 |
| 111 | $CH\equiv C-CH_2-$ | " | 1 | " | 90-92 |
| 112 | 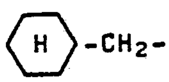 | " | 1 | 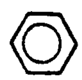 | 138 |
| 113 | 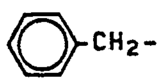 | " | 1 | " | 99 |
| 114 | H | " | 1 | 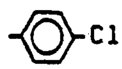 | 161-62 |
| 115 | 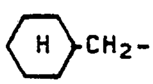 | 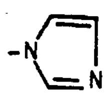 | 1 | 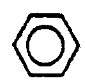 | Oel |
| 116 | n-$C_4H_9$ | 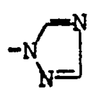 | 0 | 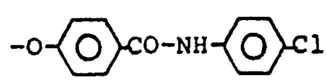 | 161-165 (x 1/2 NDS) |
| 117 | $CH_2=CH-CH_2-$ | 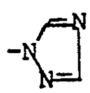 | 0 | 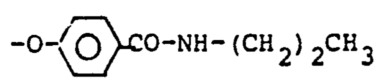 | 157-162 (x 1/2 NDS) |
| 118 | $CH=CH-CH_2-$ | 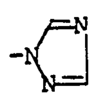 | 0 | 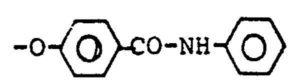 | 128 - 134 |
| 119 | n-$C_4H_9$ | 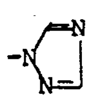 | 1 | 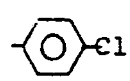 | 92 - 93 |

(Fortsetzung)

| Bsp. Nr. | $R^1$ | Az | n | $R^2$ | Schmelzpunkt (°C) Brechungsindex $n_D^{20}$ Siedepkt. (°C)/Torr |
|---|---|---|---|---|---|
| 120 | 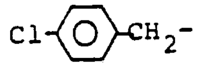 | 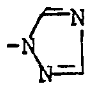 | 1 | 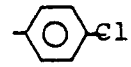 | 110 |
| 121 | 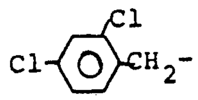 | 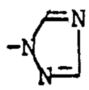 | 1 | 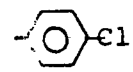 | 130 - 131 |
| 122 | 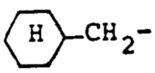 | 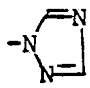 | 1 | 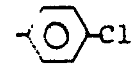 | 189 |
| 123 | 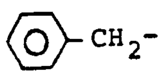 | 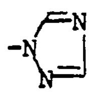 | 1 | 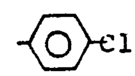 | 117 |
| 124 | n-$C_4H_9$ | 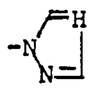 | 1 | 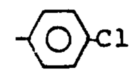 | 121 |
| 125 | 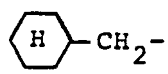 | 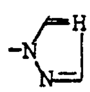 | 1 | 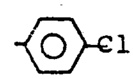 | 160 |
| 126 | H | 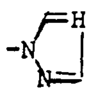 | 1 | 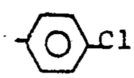 | 186 |
| 127 | 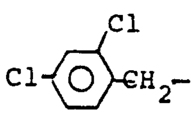 | 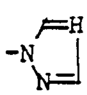 | 1 | 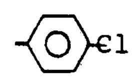 | 155 - 157 |
| 128 | 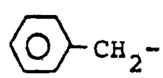 | 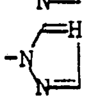 | 1 | 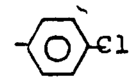 | 74 - 76 |

## Anwendungsbeispiele

Bei der Prüfung auf fungizide Wirksamkeit werden in den nachfolgenden Beispielen die nachstehend angegebenen Verbindungen als Vergleichssubstanzen eingesetzt :

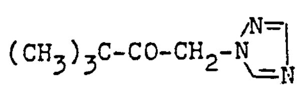
(A)

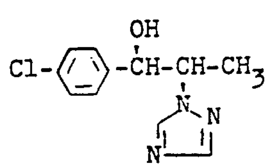
(B)

### Beispiel A

Erysiphe-Test (Gerste)/protektiv

Lösungsmittel : 100 Gewichtsteile Dimethylformamid
Emulgator : 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Sprizbelages werden die Pflanzen mit Sporen von Erysiphe graninis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutlich Überlegenheit in der Wirksamkeit gegenüber dem Stand der Technik zeigen bei diesem Test z. B. die Verbindungen gemäß folgender Herstellungsbeispiele : 18, 7, 8, 9, 5, 14 und 17.

Beispiel B

Gerstenmehltau-Test (Erysiphe graminis var. hordei)/systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22 °C und 80-90 % rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist um so wirksamer je geringer der Mehltaubefall ist.

Eine deutliche Überlegenheit in der Wirksamkeit gegenüber dem Stand der Technik zeigen bei diesem Test z. B. die Verbindungen gemäß folgender Herstellungsbeispiele : 8,9 und 17.

Beispiel C

Erysiphe-Test (Gurken)/Protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoreacearum bestäubt. Die Pflanzen werden anschließend bei 23 bis 24 °C und bei einer relativen Luftfeuchtigkeit von ca 75 % im Gewächshaus aufgestellt.

Nach 12 Tagen wird der Befall der Gurkenpflanzen bestimmt. Die erhaltenen Boniturwerte werden in ProzentBefall umgerechnet. 0% bedeutet keinen Befall, 100 % bedeutet, daß die Pflanzen vollständig befallen sind.

Eine deutliche Überlegenheit in der Wirksamkeit gegenüber dem Stand der Technik zeigen bei diesem Test z. B. die Verbindungen gemäß folgender Herstellungsbeispiele : 18, 7, 9, und 17.

Beispiel D

Wuchshemmung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösunsgmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Der erfindungsgemäße Wirkstoff (17) zeigt in diesem Test eine starke Wuchshemmung gegenüber der Kontrolle.

Beispiel E

Wuchsbeeinflussung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemäßen Wirkstoffe 5 und 17 zeigen in diesem Test eine starke Wuchsbeeinflussung gegenüber der Kontrolle.

## Ansprüche

1. Substituierte 1-Azolyl-butan-2-ole der allgemeinen Formel I

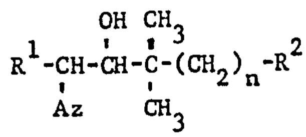

(I)

in welcher

Az für 1,2,4-Triazol-1-yl und -4-yl oder Imidazol-1-yl steht,

$R^1$ für Wasserstoff, Alkyl mit 1 bis 12 Kohlenstoffatomen, für Alkenyl und Alkinyl mit jeweils 2 bis 6 Kohlenstoffatomen steht, für gegebenfalls durch Methyl substituiertes Cycloalkyl mit 3 bis 7 Kohlenstoffatomen oder Cycloalkylalkyl mit 3 bis 7 Kohlenstoffatomen in Cycloalkylteil und 1 bis 4 Kohlenstoffatomen im Alkylteil, sowie für Phenylalkyl und Phenoxyalkyl mit jeweils 1 bis 2 Kohlenstoffatomen im Alkylteil steht, wobei die Phenylalkyl- und Phenoxyalkyl-Gruppen im Phenylteil substituiert sein können durch Halogen, Alkyl mit 1 bis 6 Kohlenstoffatomen, durch Methoxy, Methylthio, Cyclohexyl, Dimethylamino, Trifluormethyl, Trifluormethoxy, Trifluormethylthio, durch Nitro, Cyano und Alkoxycarbonyl mit 1 bis 4 Kohlenstoffatomen im Alkylteil, sowie durch jeweils gegebenenfalls halogensubstituiertes Phenyl oder Phenoxy, sowie durch die Gruppierung —CO—$NR^6R^7$,

n für 0 oder 1 steht,

$R^2$ für Phenyl steht, welches durch die bei $R^1$ genannten Phenylsubstituenten gegebenenfalls substituiert sein kann, weiterhin für Cyano, für Alkoxycarbonyl mit 1 bis 4 Kohlenstoffatomen im Alkylteil und für die Gruppierungen —X—$R^3$ und —CO—$NR^4R^5$ steht, wobei

X für Sauerstoff, Schwefel, die SO- oder $SO_2$-Gruppe steht,

$R^3$ für Alkyl mit 1 bis 4 Kohlenstoffatomen, für Phenyl und für Benzyl steht, wobei die beiden letztgenannten Gruppen durch die bei $R^1$ genannten Phenylsubstituenten gegebenenfalls substituiert sein können,

$R^4$ für Wasserstoff, Alkyl mit 1 bis 4 Kohlenstoffatomen oder für Phenyl steht, wobei letzteres durch die bei $R^1$ genannten Phenylsubstituenten substituiert sein kann,

$R^5$ für Wasserstoff und Alkyl mit 1 bis 4 Kohlenstoffatomen steht, und

$R^6$ und $R^7$ für Wasserstoff, für Alkyl mit 1 bis 4 Kohlenstoffatomen und für gegebenenfalls durch Halogen substituiertes Phenyl stehen, oder Beide zusammen mit dem verbindenden Stickstoff für einen Morpholinring stehen,

sowie deren pflanzenverträglichen Säureadditions-Salze und Metallsalz-Komplexe.

2. Verfahren zur Herstellung von substituierten 1-Azolyl-butan-2-olen der allgemeinen Formel I

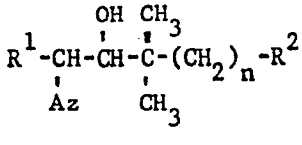

(I)

in welcher

Az für 1,2,4-Triazol-1-yl und -4-yl oder Imidazol-1-yl steht,

$R^1$ für Wasserstoff, Alkyl mit 1 bis 12 Kohlenstoffatomen, für Alkenyl und Alkinyl mit jeweils 2 bis 6 Kohlenstoffatomen steht, für gegebenenfalls durch Methyl substituiertes Cycloalkyl mit 3 bis 7 Kohlenstoffatomen oder Cycloalkylalkyl mit 3 bis 7 Kohlenstoffatomen in Cycloalkylteil und 1 bis 4 Kohlenstoffatomen im Alkylteil, sowie für Phenylalkyl und Phenoxyalkyl mit jeweils 1 bis 2 Kohlenstoffatomen im Alkylteil steht, wobei die Phenylalkyl- und Phenoxyalkyl-Gruppen im Phenylteil substituiert sein können durch Halogen, Alkyl mit 1 bis 6 Kohlenstoffatomen, durch Methoxy, Methylthio, Cyclohexyl, Dimethylamino, Trifluormethyl, Trifluormethoxy, Trifluormethylthio, durch Nitro, Cyano und Alkoxycarbonyl mit 1 bis 4 Kohlenstoffatomen im Alkylteil, sowei durch jeweils gegebenenfalls halogensubstituiertes Phenyl oder Phenoxy, sowie durch die Gruppierung —CO—$NR^6R^7$,

n für 0 oder 1 steht,

$R^2$ für Phenyl steht, welches durch die bei $R^1$ genannten Phenylsubstituenten gegebenenfalls substituiert sein kann, weiterhin für Cyano, für Alkoxycarbonyl mit 1 bis 4 Kohlenstoffatomen im Alkylteil und für die Gruppierungen —X—$R^3$ und —CO—$NR^4R^5$ steht, wobei

X für Sauerstoff, Schwefel, die SO- oder $SO_2$-Gruppe steht,

$R^3$ für Alkyl mit 1 bis 4 Kohlenstoffatomen, für Phenyl und für Benzyl steht, wobei die beiden letztgenannten Gruppen durch die bei $R^1$ genannten Phenylsubstituenten gegebenenfalls substituiert sein können,

$R^4$ für Wasserstoff, Alkyl mit 1 bis 4 Kohlenstoffatomen oder für Phenyl steht, wobei letzteres durch die bei $R^1$ genannten Phenylsubstituenten substituiert sein kann,

$R^5$ für Wasserstoff und Alkyl mit 1 bis 4 Kohlenstoffatomen steht, und

$R^6$ und $R^7$ für Wasserstoff, für Alkyl mit 1 bis 4 Kohlenstoffatomen und für gegebenenfalls durch Halogen substituiertes Phenyl stehen, oder beide zusammen mit dem verbindenden Stickstoff für einen Morpholinring stehen,

dadurch gekennzeichnet, daß man substituierte 1-Azolyl-butan-2-one der allgemeinen Formel I

$$\begin{array}{l} R^1\text{-}\underset{\displaystyle Az}{CH}\text{-}CO\text{-}\underset{\displaystyle CH_3}{C}\text{-}(CH_2)_n\text{-}R^2 \end{array} \qquad (II)$$

in welcher

Az, $R^1$, $R^2$ und n die oben angegebene Bedeutung haben,

nach bekannten Methoden in üblicher Weise reduziert und gegebenenfalls anschließend eine Säure oder ein Metallsalz addiert.

3. Pflanzenschutzmittel, gekennzeichnet durch einen Gehalt an mindestens einem substituierten 1-Azolyl-butan-2-ol der allgemeinen Formel I in Ansprüchen 1 und 2.

4. Verwendung von substituierten 1-Azolyl-butan-2-olen der allgemeinen Formel I in Ansprüchen 1 und 2 als Pflanzenschutzmittel.

5. Verwendung von substituierten 1-Azolyl-butan-2-olen der allgemeinen Formel I in Ansprüchen 1 und 2 zur Bekämpfung von phytopathogenen Pilzen.

6. Verwendung von substituierten 1-Azolyl-butan-2-olen der allgemeinen Formel I in Ansprüchen 1 und 2 zur Regulierung des Pflanzenwachstums.

7. Verfahren zur Herstellung von Pflanzenschutzmitteln, dadurch gekennzeichnet, daß man substituierte 1-Azolyl-butan-2-ole der allgemeinen Formel I in Ansprüchen 1 und 2 mit Streckmitteln und/oder oberflächenaktiven Mitteln vermischt.

## Claims

1. Substituted 1-azolyl-butan-2-ols of the general formula I

$$R^1\text{-}\underset{\displaystyle Az}{CH}\text{-}\overset{\displaystyle OH}{CH}\text{-}\overset{\displaystyle CH_3}{\underset{\displaystyle CH_3}{C}}\text{-}(CH_2)_n\text{-}R^2 \qquad (I)$$

in which

Az represents 1,2,4-triazol-1-yl or -4-yl or imidazol-1-yl,

$R^1$ represents hydrogen, alkyl with 1 to 12 carbon atoms, alkenyl or alkinyl with in each case 2 to 6 carbon atoms, optionally methyl-substituted cycloalkyl with 3 to 7 carbon atoms or cycloalkylalkyl with 3 to 7 carbon atoms in the cycloalkyl part and 1 to 14 carbon atoms in the alkyl part, or phenylalkyl or phenoxyalkyl with in each case 1 to 2 carbon atoms in the alkyl part, it being possible for the phenylalkyl and phenoxyalkyl groups to be substituted in the phenyl part by halogen, alkyl with 1 to 6 carbon atoms, by methoxy, methylthio, cyclohexyl, dimethylamino, trifluoromethyl, trifluoromethoxy, trifluoromethyl-

thio, by nitro, cyano or alkoxycarbonyl with 1 to 4 carbon atoms in the alkyl part, or by phenyl or phenoxy which are each optionally halogen-substituted, or by the grouping —CO—$NR^6R^7$,

n represents 0 or 1,

$R^2$ represents phenyl, which can optionally be substituted by the phenyl substituents mentioned under $R^1$, or represents cyano, alkoxycarbonyl with 1 to 4 carbon atoms in the alkyl part or the groupings —X—$R^3$ or —CO—$NR^4R^5$, wherein X represents oxygen, sulphur or the SO or $SO_2$ group,

$R^3$ represents alkyl with 1 to 4 carbon atoms, phenyl or benzyl, it being possible for the latter two groups to be optionally substituted by the phenyl substituents mentioned under $R^1$,

$R^4$ represents hydrogen, alkyl with 1 to 4 carbon atoms or phenyl, it being possible for the latter to be substituted by the phenyl substituents mentioned under $R^1$,

$R^5$ represents hydrogen or alkyl with 1 to 4 carbon atoms, and

$R^6$ and $R^7$ represent hydrogen, alkyl with 1 to 4 carbon atoms or optionally halogen-substituted phenyl, or both, together with the linking nitrogen, represent a morpholine ring,

and acid addition salts and metal salt complexes thereof which are tolerated by plants.

2. Process for the preparation of substituted 1-azolyl-butan-2-ols of the general formula I

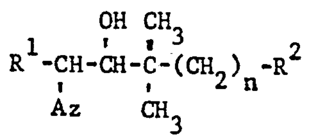

(I)

in wich

Az represents 1,2,4-triazol-1-yl or -4-yl or imidazol-1-yl,

$R^1$ represents hydrogen, alkyl with 1 to 12 carbon atoms alkenyl or alkinyl with in each case 2 to 6 carbon atoms, optionally methyl-substituted cycloalkyl with 3 to 7 carbon atoms or cycloalkylalkyl with 3 to 7 carbon atoms in the cycloalkyl part and 1 to 4 carbon atoms in the alkyl part, or phenylalkyl or phenoxyalkyl with in each case 1 to 2 carbon atoms in the alkyl part, it being possible for the phenylalkyl and phenoxyalkyl groups to be substituted in the phenyl part by halogen, alkyl with 1 to 6 carbon atoms, by methoxy, methylthio, cyclohexyl, dimethylamino, trifluoromethyl, trifluoromethoxy, trifluoromethylthio, by nitro, cyano or alkoxycarbonyl with 1 to 4 carbon atoms in the alkyl part, or by phenyl or phenoxy which are each optionally halogen-substituted, or by the grouping —CO—$NR^6R^7$,

n represents 0 or 1,

$R^2$ represents phenyl, which can optionally be substituted by the phenyl substituents mentioned under $R^1$, or represents cyano, alkoxycarbonyyl with 1 to 4 carbon atoms in the alkyl part or the groupings —X—$R^3$ or —CO—$NR^4R^5$, wherein

X represents oxygen, sulphur or the SO or $SO_2$ group,

$R^3$ represents alkyl with 1 to 4 carbon atoms, phenyl or benzyl, it being possible for the latter two groups to be optionally substituted by the phenyl substituents mentioned under $R^1$,

$R^4$ represents hydrogen, alkyl with 1 to 4 carbon atoms or phenyl, it being possible for the latter to be substituted by the phenyl substituents mentioned under $R^1$,

$R^5$ represents hydrogen or alkyl with 1 to 4 carbon atoms, and

$R^6$ and $R^7$ represent hydrogen, alkyl with 1 to 4 carbon atoms or optionally halogen-substituted phenyl, or both, together with the linking nitrogen, represent a morpholine ring,

characterised in that substituted 1-azolyl-butan-2-ones of the general formula II

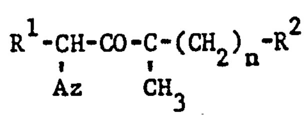

(II)

in which

Az, $R^1$, $R^2$ and n have the abovementioned meaning, are reduced by known methods in the customary manner and, if appropriate, an acid or a metal salt is then added on.

3. Plant protection agents, characterised in that they contain at least one substituted 1-azolyl-butan-2-ol of the general fomula I in Claims 1 and 2.

4. Use of substituted 1-azolyl-butan-2-ols of the general formula I in Claims 1 and 2 as plant protection agents.

5. Use of substituted 1-azolyl-butan-2-ols of the general formula I in Claims 1 and 2 for combating phytopathogenic fungi.

6. Use of substituted 1-azolyl-butan-2-ols of the general formula I in Claims 1 and 2 for regulating plant growth.

7. Process for the preparation of plant protection agents, characterised in that substituted 1-azolyl-butan-2-ols of the general formula I in Claims 1 and 2 are mixed with extenders and/or surface-active agents.

**Revendications**

1. 1-azolyl-butane-2-ols substitués de formule générale I

$$R^1-\underset{Az}{CH}-\overset{OH}{CH}-\overset{CH_3}{\underset{CH_3}{C}}-(CH_2)_n-R^2 \qquad (I)$$

dans laquelle

Az représente un groupe 1,2,4-triazol-1-yle et -4-yle ou imidazol-1-yle,

$R^1$ représente de l'hydrogène, un groupe alkyle ayant 1 à 12 atomes de carbone, alcényle et alcynyle ayant chacun 2 à 6 atomes de carbone, un groupe cycloalkyle ayant 3 à 7 atomes de carbone et éventuellement substitué par du méthyle, ou cycloalkylalkyle ayant 3 à 7 atomes de carbone dans la partie cycloalkyle et 1 à 4 atomes de carbone dans la partie alkyle, ainsi qu'un groupe phénylalkyle et phénoxyalkyle ayant chacun 1 ou 2 atomes de carbone dans le partie alkyle, les groupes phénylalkyle et phénoxyalkyle de la partie alkyle, les groupes phénylalkyle et phénoxyalkyle de la partie phényle pouvant être substitués par de l'halogène, un groupe alkyle ayant 1 à 6 atomes de carbone, par un groupe méthoxy, méthylthio, cyclohexyle, diméthylamino, trifluorométhyle, trifluorométhoxy, trifluorométhylthio, par un groupe nitro, cyano et alcoxycarbonyle ayant 1 à 4 atomes de carbone dans la partie alkyle, ainsi que par un groupe phényle ou phénoxy chacun éventuellement substitué par un halogène, ainsi que par le groupement —CO—$NR^6R^7$,

n vaut 0 ou 1,

$R^2$ représente un groupe phényle, qui peut être éventuellement substitué par les substituants du groupe phényle cités pour $R^1$, en outre un goupe cyano, alcoxycarbonyle ayant 1 à 4 atomes de carbone dans la partie alkyle et les groupements —X—$R^3$ et —CO—$NR^4R^5$, où

X représente l'oxygène, le soufre, le groupe —SO ou —$SO_2$,

$R^3$ représente un groupe alkyle ayant 1 à 4 atomes de carbone, phényle ou benzyle, ces deux derniers groupes cités pouvant éventuellement être substitués par les substituants du groupe phényle cités pour $R^1$,

$R^4$ représente l'hydrogène, un groupe alkyle ayant 1 à 4 atomes de carbone ou phényle, ce dernier pouvant être substitué éventuellement par les substituants du groupe phényle cités pour $R^1$,

$R^5$ représente un atome d'hydrogène et un groupe alkyle ayant 1 à 4 atomes de carbone, et

$R^6$ et $R^7$ représentent de l'hydrogène, un groupe alkyle ayant 1 à 4 atomes de carbone et un groupe phényle éventuellement substitué par de l'halogène, ou les deux radicaux, pris avec l'atome d'azote auquel ils sont reliés, forment un noyau morpholine,

ainsi que leurs sels d'addition d'acides et complexes de sels de métaux tolérables par les plantes.

2. Procédé de préparation de 1-azolyl-butane-2-ols substitués de formule générale I

$$R^1-\underset{Az}{CH}-\overset{OH}{CH}-\overset{CH_3}{\underset{CH_3}{C}}-(CH_2)_n-R^2 \qquad (I)$$

dans laquelle

Az représente un groupe 1,2,4-triazol-1-yle et -4-yle ou imidazol-1-yle,

$R^1$ représente de l'hydrogène, un groupe alkyle ayant 1 à 12 atomes de carbone, alcényle et alcynyle ayant chacun 2 à 6 atomes de carbone, cycloalkyle ayant 3 à 7 atomes de carbone, éventuellement substitués par un groupe méthyle, ou cycloalkylalkyle ayant 3 à 7 atomes de carbone dans la partie cycloalkyle et 1 à 4 atomes de carbone dans la partie alkyle ainsi que phénylalkyle et phénoxyalkyle ayant chacun 1 à 2 atomes de carbone dans la partie alkyle, les groupes phénylalkyle et phénoxyalkyle pouvant être substitués dans la partie phényle par de l'halogène, un groupe alkyle ayant 1 à 6 atomes de carbone, par un groupe méthoxy, méthylthio, cyclohexyle, diméthylamino, trifluorométhyle, trifluorométhoxy, trifluorométhylthio, par un groupe nitro, cyano et alcoxycarbonyle ayant 1 à 4 atomes de carbone dans la partie alkyle, ainsi que par un groupe phényle ou phénoxy éventuellement chacun substitués par de l'halogène, ainsi que par le groupement —CO—$NR^6R^7$,

n vaut 0 ou 1,

$R^2$ représente un groupe phényle, qui peut éventuellement être substitué par les substituants du groupe phényle cités pour $R^1$, ainsi qu'un groupe cyano, alcoxycarbonyle ayant 1 à 4 atomes de carbone dans la partie alkyle et les groupements —X—$R^3$ et —CO—$NR^4R^5$, où

X représente de l'oxygène ou du soufre ou le groupe —SO ou —$SO_2$,

$R^3$ représente un groupe alkyle ayant 1 à 4 atomes de carbone, phényle et benzyle, ces deux derniers groupes pouvant être éventuellement substitués par des substituants du groupe phényle cités pour $R^1$,

$R^4$ représente de l'hydrogène, un groupe alkyle ayant 1 à 4 atomes de carbone ou phényl, ce dernier pouvant être substitué par les substituants du groupe phényl cités pour $R^1$,

$R^5$ représente de l'hydrogène et un groupe alkyle ayant 1 à 4 atomes de carbone, et

$R^6$ et $R^7$ représentent de l'hydrogène, un groupe alkyle ayant 1 à 4 atomes de carbone et un groupe phényle éventuellement substitué par de l'halogène, ou bien les deux radicaux, pris avec l'atome d'azote qui les relie, forment un noyau morpholine,

procédé caractérisé en ce qu'on réduit de façon usuelle, selon des méthodes connues, des 1-azolyl-butane-2-ones substituées, de formule générale II

$$R^1\text{-}\underset{\displaystyle Az}{CH}\text{-}CO\text{-}\underset{\displaystyle CH_3}{C}\text{-}(CH_2)_n\text{-}R^2 \qquad \text{(II)}$$

dans laquelle

Az, $R^1$, $R^2$ et n ont le sens indiqué ci-dessus

et éventuellement, on fixe ensuite par addition un acide ou un sel de métal.

3. Agent de protection des plantes, caractérisé par une teneur en au moins un 1-azolyl-butane-2-ol substitué de formule générale I selon les revendications 1 et 2.

4. Utilisation des 1-azolyl-butane-2-ols substitués, répondant à la formule générale I selon les revendications 1 et 2, comme produits phytosanitaires.

5. Utilisation des 1-azolyl-butane-2-ols de formule générale I selon les revendications 1 et 2 pour la lutte contre les champignons phytopathogènes.

6. Utilisation des 1-azolyl-butane-1-ols substitués de formule générale I selon les revendications 1 et 2, pour la régulation de la croissance des plantes.

7. Procédé de préparation de produits phytosanitaires, caractérisé en ce qu'on mélange des 1-azolyl-butane-2-ols substitués, de formule générale I selon les revendications 1 et 2, avec des agents d'allongement et/ou des agents tensioactifs.